# EUROPÄISCHE PATENTANMELDUNG

(11) **EP 4 042 879 A1**
(43) Veröffentlichungstag der Anmeldung: **17.08.2022**
(21) Anmeldenummer: 22163412.4
(22) Anmeldetag: 13.08.2020
(51) Int. Cl.: A23L 29/00, A23L 29/212, A23L 29/238, A23L 29/262, A23L 29/269, C08L 3/02, C08L 5/00, C08L 5/12, A47G 21/18, A47G 21/00, A61B 13/00, A61C 17/00, B29C 48/00

(54) **KOMPOSTIERBARE MEDIZINISCHE BEDARFSARTIKEL AUS PFLANZLICHER STÄRKE UND PFLANZLICHEM DICKUNGS- ODER GELIERMITTEL UND VERFAHREN ZUR HERSTELLUNG DERSELBEN**

(30) Priorität: 13.08.2019 DE 102019212126; 11.05.2020 EP 20173968
(62) Teilanmeldung aus: 20764951.8
(71) Anmelder: Hope Tree International GmbH, 83607 Holzkirchen (DE)
(72) Erfinder: DINZINGER, Lambert Dustin, 82538 Geretsried-Gelting (DE)
(74) Vertreter: Grund, Martin

(57) **Zusammenfassung**

Die vorliegende Erfindung betrifft kompostierbare und biologisch abbaubare medizinische Bedarfsartikel wie etwa Ohrenstäbchen, Mundspatel oder Einmalzahnbürsten. Die medizinischen Bedarfsartikel der vorliegenden Erfindung umfassen pflanzliche Stärke und pflanzliche Dickungs- und Geliermittel und können ferner Zellstoff/Holzstoff, Wachs, Öl, und /oder Glycerin umfassen. Ferner können die medizinischen Bedarfsartikel der vorliegenden Erfindung eine äußere Beschichtung umfassen, welche bevorzugt Wachs und/oder ein Biopolymer, bevorzugt Kautschuk umfasst. Die vorliegende Erfindung betrifft zudem Herstellungsverfahren für kompostierbare, biologisch abbaubare medizinische Bedarfsmittel.

## Beschreibung

### Gebiet der Erfindung

Die vorliegende Erfindung liegt auf dem Gebiet von biologisch abbaubaren Hilfsmitteln zum Essen oder zum Trinken umfassend Trinkhalme / Strohhalme und Besteck und Verfahren zu deren Herstellung. Unter Verwendung von pflanzlicher Stärke und pflanzlichem Dickungs- oder Geliermittel können recycelbare und biologisch abbaubare Hilfsmittel zum Essen oder zum Trinken hergestellt werden. Die Zugabe von Zellstoff, Holzstoff, Wachs, insbesondere Carnaubawachs, und/oder Bio-Polymeren, insbesondere Naturlatex kann den Hilfsmitteln zum Essen oder zum Trinken weitere vorteilhafte Eigenschaften verleihen. Hierbei können Hilfsmittel zum Essen oder zum Trinken in halb-transparenter oder opaker Ausführungsform hergestellt werden. Optional kann durch Zugabe von Lebensmittelfarbe die Farbe modifiziert werden.

Dieselben Bestandteile wie sie für die Herstellung von Hilfsmittel zum Essen oder zum Trinken verwendet werden, eignen sich auch zur Herstellung von medizinischen Bedarfsartikeln wie Mundspatel oder Ohrenstäbchen. Daher betrifft die vorliegende Erfindung auch biologisch abbaubare medizinische Bedarfsartikel und deren Herstellung.

### Hintergrund der Erfindung

Allein in Deutschland werden täglich tausende Trinkhalme / Strohhalme und Einweg-Besteck verbraucht. Herkömmliche Einweg-Trinkhalme und Einweg-Bestecke sind weder recycle- noch kompostierbar, sie können nur verbrannt oder unter enormem Kosten- und Energieaufwand verwertet werden und schaden so dem Erhalt unseres Ökosystems. Infolgedessen ist der Bedarf an einer umweltfreundlichen, biologisch abbaubaren und kompostierbaren Alternative zu herkömmlichen Trinkhalmen und Besteck aber auch anderen Hilfsmitteln zum Essen oder zum Trinken wie kompostierbare Lollistiele/Lollisticks enorm groß. Auch bei medizinischen Bedarfsartikeln wie Ohrenstäbchen, oder Wattestäbchen zur beispielsweise Probenentnahme ist eine biologisch abbaubare und kompostierbare Lösung wünschenswert.

Eine solche Alternative bieten die Hilfsmittel zum Essen oder zum Trinken der vorliegenden Erfindung, sowie die Verfahren zu deren Herstellung. Während herkömmliche Hilfsmittel zum Essen oder zum Trinken wie Trinkhalme und Besteck aus Plastik bestehen, bestehen Hilfsmittel zum Essen oder zum Trinken der vorliegenden Erfindung aus rein pflanzlichen Materialien, wie pflanzlicher Stärke, pflanzlichem Dickungs- oder Geliermittel, Zellstoff, Holzstoff und Carnaubawachs. Die z.B. Trinkhalme und das Besteck sind somit nicht nur leicht zu recyceln bzw. zu kompostieren, sie werden auch aus nachwachsenden Rohstoffen produziert, die eine positive CO₂-Bilanz aufweisen. Daher bilden die Hilfsmittel zum Essen oder zum Trinken der vorliegenden Erfindung eine umweltfreundliche Alternative zu gebräuchlichen Lösungen wie Einweg-Trinkhalmen aus Plastik.

Dasselbe Material kann auch für die Herstellung medizinischer Bedarfsmittel verwendet werden. Daher bilden auch die medizinischen Bedarfsmittel der vorliegenden Erfindung eine umweltfreundliche Alternative zu gebräuchlichen Lösungen wie Ohrenstäbchen aus Plastik.

### Zusammenfassung der Erfindung

Die vorliegende Erfindung bezieht sich auf Hilfsmittel zum Essen und zum Trinken sowie auf medizinische Bedarfsmittel und Verfahren zu deren Herstellung.

Die Hilfsmittel zum Essen und zum Trinken der vorliegenden Erfindung sind kompostierbar.

Die Hilfsmittel zum Essen und zum Trinken der vorliegenden Erfindung sind innerhalb eines Zeitraums von 4-8 Wochen, bevorzugt 4 Wochen kompostierbar.

Die Hilfsmittel zum Essen und zum Trinken der vorliegenden Erfindung sind innerhalb von 50 Tagen biologisch abbaubar.

Die vorliegende Erfindung bezieht sich auf Hilfsmittel zum Essen oder zum Trinken umfassend pflanzliche Stärke und pflanzliches Dickungs- oder Geliermittel.

In manchen Ausführungsformen können die Hilfsmittel zum Essen oder zum Trinken weitere Bestandteile aufweisen. Die Hilfsmittel zum Essen oder zum Trinken gemäß der vorliegenden Erfindung können ferner Zellstoff und/oder Holzstoff umfassen.

Die Hilfsmittel zum Essen oder zum Trinken gemäß der vorliegenden Erfindung können ferner Wachs, bevorzugt Carnaubawachs oder Sojawachs umfassen.

Die Hilfsmittel zum Essen oder zum Trinken gemäß der vorliegenden Erfindung können ferner Öl, bevorzugt Nussöl oder Sonnenblumenöl umfassen.

Die Hilfsmittel zum Essen oder zum Trinken gemäß der vorliegenden Erfindung können ferner Glycerin umfassen.

Die Hilfsmittel zum Essen oder zum Trinken gemäß der vorliegenden Erfindung können ferner eine äußere Beschichtung umfassend Carnaubawachs, und/oder Rapswachs, und/oder Paraffin, oder Mischungen davon umfassen. Der Beschichtung wurde vorzugsweise ein oder mehrere Biopolymere, insbesondere Naturlatex hinzugefügt. Besonders bevorzugt ist eine Wachsschicht umfassend Carnaubawachs, die zusätzlich Naturlatex enthält.

Die Beschichtung der Hilfsmittel zum Essen oder zum Trinken gemäß der vorliegenden Erfindung kann ferner Hilfsstoffe und/oder anti-stick Zusätze umfassen.

Die äußere Wachsschicht, im Sinne der vorliegenden Erfindung, umfasst auch die Beschichtung innenliegender Oberflächen, wie sie beispielsweise beim Trinkhalm gegeben sind.

In manchen Ausführungsformen umfasst die pflanzliche Stärke Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote.

In manchen Ausführungsformen ist die pflanzliche Stärke Weizenstärke, Kartoffelstärke, Maisstärke, Tapiokastärke oder eine Mischung davon.

In manchen Ausführungsformen umfasst das pflanzliche Dickungs- oder Geliermittel Agar-Agar, Guarkernmehl, Xanthan oder eine Mischung aus Guarkernmehl und Xanthan.

In manchen Ausführungsformen ist das pflanzliche Dickungs- oder Geliermittel eine Mischung aus Guarkernmehl und Xanthan.

In manchen Ausführungsformen umfasst das Hilfsmittel zum Essen oder zum Trinken
40-50% pflanzliche Stärke,
35-45% Guarkernmehl und
1-5% Xanthan.

In manchen Ausführungsformen umfasst das Hilfsmittel zum Essen oder zum Trinken
40-50% pflanzliche Stärke,
35-45% Guarkernmehl
1-5% Xanthan und
eine Wachs- und/oder Naturlatex-basierte Beschichtung.

Das Hilfsmittel zum Essen oder zum Trinken gemäß der vorliegenden Erfindung kann ein Trinkhalm, ein Becher, ein Besteck oder ein Lollistiel/Lollitstick sein. Das Hilfsmittel zum Trinken der vorliegenden Erfindung ist bevorzugt ein Trinkhalm. Das Hilfsmittel zum Essen der vorliegenden Erfindung ist bevorzugt ein Lollistiel/Lollistick.

In einer konkreten Ausführung umfasst das Hilfsmittel zum Essen oder zum Trinken
40-50% Stärke,
35-45% Guarkernmehl,
1-5% Xanthan,
Farbstoff, und eine
Naturlatex-basierte Beschichtung,
wobei die Beschichtung aus 5% Carnaubawachs, 2% Glycerin, 93% Naturlatex besteht und wobei das Hilfsmittel ein kompostierbarer Trinkhalm ist.

Die vorliegende Erfindung bezieht sich auch auf medizinische Bedarfsartikel.

Die medizinischen Bedarfsartikel der vorliegenden Erfindung sind kompostierbar.

Die medizinischen Bedarfsartikel der vorliegenden Erfindung sind innerhalb eines Zeitraums von 4-8 Wochen, bevorzugt 4 Wochen kompostierbar.

Die medizinischen Bedarfsartikel der vorliegenden Erfindung sind innerhalb von 50 Tagen biologisch abbaubar.

Die vorliegende Erfindung bezieht sich auch auf medizinische Bedarfsartikel umfassend pflanzliche Stärke und pflanzliches Dickungs- oder Geliermittel.

In manchen Ausführungsformen können die medizinischen Bedarfsartikel weitere Bestandteile aufweisen.

Die medizinischen Bedarfsartikel gemäß der vorliegenden Erfindung können ferner Zellstoff und/oder Holzstoff umfassen.

Die medizinischen Bedarfsartikel gemäß der vorliegenden Erfindung können ferner Wachs, bevorzugt Carnaubawachs oder Sojawachs umfassen.

Die medizinischen Bedarfsartikel gemäß der vorliegenden Erfindung können ferner Öl, bevorzugt Nussöl oder Sonnenblumenöl umfassen.

Die medizinischen Bedarfsartikel gemäß der vorliegenden Erfindung können ferner Glycerin umfassen.

Die medizinischen Bedarfsartikel gemäß der vorliegenden Erfindung können ferner eine äußere Beschichtung umfassend Carnaubawachs, und/oder Rapswachs, und/oder Paraffin, oder Mischungen davon umfassen. Die Beschichtung umfasst vorzugsweise Biopolymer, insbesondere Naturlatex. Besonders bevorzugt ist eine Beschichtung umfassend Carnaubawachs, die zusätzlich Naturlatex enthält.

In manchen Ausführungsformen umfasst die pflanzliche Stärke Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote.

In manchen Ausführungsformen ist die pflanzliche Stärke Weizenstärke, Kartoffelstärke, Maisstärke, Tapiokastärke oder eine Mischung davon.

In manchen Ausführungsformen umfasst das pflanzliche Dickungs- oder Geliermittel Agar-Agar, Guarkernmehl, Xanthan oder eine Mischung aus Guarkernmehl und Xanthan.

In manchen Ausführungsformen ist das pflanzliche Dickungs- oder Geliermittel eine Mischung aus Guarkernmehl und Xanthan.

In manchen Ausführungsformen umfasst der medizinische Bedarfsartikel
40-50% pflanzliche Stärke,
35-45% Guarkernmehl und
1-5% Xanthan.

In manchen Ausführungsformen umfasst der medizinische Bedarfsartikel
40-50% pflanzliche Stärke,
35-45% Guarkernmehl
1-5% Xanthan und
eine Wachs-basierte Beschichtung.

In manchen Ausführungsformen ist der medizinische Bedarfsartikel ein Wattestäbchen, ein Ohrenstäbchen, ein Mundspatel oder eine Einmalzahnbürste. Bevorzugt ist der medizinische Bedarfsartikel ein Ohrenstäbchen.

Die vorliegende Erfindung betrifft ferner ein Verfahren zur Herstellung von kompostierbaren Hilfsmitteln zum Essen oder zum Trinken. Außerdem betrifft die vorliegende Erfindung ein Verfahren zur Herstellung von kompostierbaren medizinischen Bedarfsartikeln.

Die vorliegende Erfindung bezieht sich auf ein Verfahren zur Herstellung von Hilfsmitteln zum Essen oder Trinken oder medizinischen Bedarfsartikeln, wobei das Verfahren die folgenden Schritte umfasst:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung umfassend pflanzliche Stärke und pflanzliches Dickungs- und Geliermittel und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu einem Hilfsmittel zum Essen oder Trinken oder zu einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Emulsion umfassend Carnaubawachs, und/oder Rapswachs, und/oder Paraffin, oder Mischungen davon. Besonders bevorzugt ist eine Emulsion umfassend Carnaubawachs, die zusätzlich Naturlatex enthält.

Die vorliegende Erfindung bezieht sich auch auf ein Verfahren zur Herstellung von Hilfsmitteln zum Essen oder Trinken oder medizinischen Bedarfsartikeln, wobei das Verfahren die folgenden Schritte umfasst:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung umfassend
      50-70% pflanzliche Stärke und
      30-50% pflanzliches Dickungs- und Geliermittel
      und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile.
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Emulsion umfassend Carnaubawachs, und/oder Rapswachs, und/oder Paraffin, oder Mischungen davon. Besonders bevorzugt ist eine Emulsion umfassend Carnaubawachs, die zusätzlich Naturlatex enthält.

In manchen Ausführungsformen umfasst die die pflanzliche Stärke in der Mischung in Schritt a.1 Weizenstärke. Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote.

In manchen Ausführungsformen ist die pflanzliche Stärke in der Mischung in Schritt a.1 Weizenstärke, Kartoffelstärke, Maisstärke, Tapiokastärke oder eine Mischung davon.

In manchen Ausführungsformen umfasst das pflanzliche Dickungs- oder Geliermittel in der Mischung in Schritt a.1 Agar-Agar, Guarkernmehl, Xanthan oder eine Mischung aus Guarkernmehl und Xanthan.

In manchen Ausführungsformen ist das pflanzliche Dickungs- oder Geliermittel in der Mischung in Schritt a.1 eine Mischung aus Guarkernmehl und Xanthan.

In manchen Ausführungsformen umfassen die flüssigen Bestandteile aus Schritt a.2 ferner Öl, bevorzugt Nussöl oder Sonnenblumenöl, Glycerin und/oder eine Wachsemulsion.

In einer bevorzugten Ausführungsform umfasst das Verfahren zur Herstellung von Hilfsmitteln zum Essen oder Trinken oder medizinischen Bedarfsartikeln die folgenden Schritte:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung umfassend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit Emulsion umfassend Carnaubawachs, und/oder Rapswachs, und/oder Paraffin, oder Mischungen davon. Besonders bevorzugt ist eine Emulsion umfassend Carnaubawachs, die zusätzlich Naturlatex enthält.

In einer bevorzugten Ausführungsform besteht die Emulsion im optionalen Schritt d. des Herstellungsverfahrens entweder aus 32,5% Carnaubawachs, 17,5% Paraffin und 50% Wasser oder aus 50% Carnaubawachs und 50% Wasser (Wachs-basierte Beschichtung), oder aus 5% Carnaubawachs, 45% Kautschuk und 50% Wasser, oder 5% Carnaubawachs und 95% Naturlatex (Naturlatex-basierte Beschichtung). Optional können der Emulsion Hilfsstoffe wie Farbstoffe oder Geruchsneutralisatoren und/oder "anti-stick" Zusätze, wie auch Glycerin zugegeben werden.

### Vorteile der Erfindung

Durch die Verwendung der pflanzlichen Stärke und pflanzlichen Dickungs- und Geliermittel sind die Hilfsmittel zum Essen oder zum Trinken und die medizinischen Bedarfsartikel der vorliegenden Erfindung im Gegensatz zu herkömmlichen Einweg-Lösungen wie Plastiktrinkhalme, Plastikbesteck oder Plastik-Ohrenstäbchen recycelbar, vollständig biologisch abbaubar und auch kompostierbar. So wurde die Kompostierdauer eines erfindungsgemäßen Trinkhalms, welcher hergestellt wurde aus einer Mischung umfassend 50% Stärke, 45% Guarkernmehl 5% Xanthan und bezogen auf das Nettogewicht dieser Bestandteile 2% Carnaubawachsemulsion, 40% Wasser und 4% Öl von einer zuständigen Behörde (DEKRA) als Lebensmittelecht getestet. Ferner wurde dieser Trinkhalm auf seine Kompostierbarkeit getestet. Die Testung ergab eine Kompostierdauer des Trinkhalms von unter 4 Wochen in der Intensivrotte, und von 27 Tagen bei Trockenvergärung. Dieser Wert liegt weit unter dem Wert alternativer Jegliches Hilfsmittel oder jeglicher Bedarfsartikel der vorliegenden Erfindung umfassend diese Bestandteile sind demnach innerhalb dieser Zeit kompostierbar. Trinkhalme. Eine optionale Beschichtung verlängert den Kompostiervorgen um nur wenige Tage, sodass auch beschichtete Hilfs- oder Bedarfsmittel der vorliegenden Erfindung innerhalb 4-8 Wochen kompostierbar sind. Ferner sind die Hilfsmittel und die Bedarfsartikel der vorliegenden Erfindung gemäß EN 13432, Version 2000-12 kompostierbar.

In manchen Ausführungsformen umfassen die Hilfsmittel zum Essen oder zum Trinken und die medizinischen Bedarfsartikel der Erfindung Zellstoff. Dies erhöht die mechanische Stabilität der Hilfsmittel zum Essen oder zum Trinken und der medizinischen Bedarfsartikel und kann so das Abknicken oder Zerreisen dieser verhindern. Die Hilfsmittel und die Bedarfsartikel der vorliegenden Erfindung erhalten bis zu 15% Zellstoff. Ein höherer Zellstoff-Gehalt würde die Hilfsmittel oder Bedarfsmittel zu spröde machen. Besonders gute mechanische Eigenschaften ergeben sich bei einem Zellstoff-Gehalt von 2-10%.

In manchen Ausführungsformen umfassen die Hilfsmittel zum Essen oder zum Trinken und die medizinischen Bedarfsartikel der Erfindung Holzstoff. Wie Zellstoff erhöht auch Holzstoff die mechanische Stabilität. Im Vergleich zu Holzstoff hat Zellstoff den Vorteil, dass die Hilfsmittel oder Bedarfsartikel eine höhere Transparenz aufweisen.

In manchen Ausführungsformen umfassen die Hilfsmittel zum Essen oder zum Trinken und die medizinischen Bedarfsartikel der Erfindung Wachs, bevorzugt Carnaubawachs oder Sojawachs. Wachs erhöht die Resistenz der Hilfsmittel zum Essen oder zum Trinken und der medizinischen Bedarfsartikel gegenüber Wasser und Öl. Das höchst widerstandfähige Carnaubawachs kann die Stabilität während der Benutzung in einer Flüssigkeit um mindestens 10%, mindestens 20%, mindestens 30% oder sogar um mehr als 30% steigern. Auch die Verwendung von Sojawachs zeigt diesen Effekt und ist bevorzugt.

In manchen Ausführungsformen umfassen die Hilfsmittel zum Essen oder zum Trinken und die medizinischen Bedarfsartikel der Erfindung Öl, bevorzugt Nussöl. Dies verbessert die Fließeigenschaften der Rohmasse für die Hilfsmittel zum Essen oder zum Trinken und der medizinischen Bedarfsartikel während der Herstellung.

Der Anteil an pflanzlichen Dickungs- und Geliermittel beeinflusst ebenso die Transparenz des Materials. Je höher der Anteil der pflanzlichen Dickungs- oder Geliermittel, desto höher wird die Transparenz.

In manchen Ausführungsformen umfassen die Hilfsmittel zum Essen oder zum Trinken und die medizinischen Bedarfsartikel der Erfindung Magnesiumstearat und/oder Calciumstearat und/oder Zinkstearat. Magnesium- und Calciumstearat werden hierbei als Schmiermittel, Antihaftmittel und Geliermittel für eine verbesserte Fließeigenschaft der Rohmasse während der Herstellung verwendet. Die Zugabe von Zinkstearat verbessert die Aufnahme der Rohmasse in beim Herstellungsverfahren verwendeten Vorrichtungen, z.B. das Einziehen der Rohmasse mittels einer Plastifizierschnecke. In manchen Ausführungsformen wird im Herstellungsverfahren ein Teil des zugegebenen Wassers durch Glycerin E422 ersetzt, um die Beweglichkeit bzw. die Biegeeigenschaft der erfindungsgemäßen Hilfsmittel zum Essen oder zum Trinken und der medizinischen Bedarfsartikel zu erhöhen.

Trinkhalme mit einer äußeren und inneren Beschichtung aus Carnaubawachs und Kautschuk sind im Wasser stehend bis zu sechs Tage stabil, gleichzeitig aber weiterhin biologisch abbaubar und kompostierbar: im Gartenkompost wurden die Trinkhalme der Erfindung in weniger als 50 Tagen, vorzugsweise weniger als 30 Tagen biologisch abgebaut. In industriellen Kompostanlagen wurden die Trinkhalme in weniger als 14 Tagen nicht mehr nachgewiesen und waren somit biologisch abgebaut. Dies trifft somit auch auf die anderen erfindungsgemäßen Hilfsmittel mit der gleichen oder ähnlichen Komposition zu.

Auch wurden die erfindungsgemäßen Trinkhalme einer Prüfung mit Lebensmittelkontakt bei einer offiziellen Prüfstelle (DEKRA e.V.) unterzogen. Diese Prüfung wurde bestanden, weder Schwermetalle, noch Pestizide, noch andere Schadstoffe wie Glyphosat wurden nachgewiesen. Folglich treffen diese ermittelten Prüfergebnisse auch auf die anderen erfindungsgemäßen Hilfsmittel mit der gleichen oder ähnlichen Komposition zu.

Ferner verlieht die erfindungsgemäße Beschichtung dem Trinkhalm beziehungsweise den Hilfsmitteln weitere vorteilhafte Eigenschaften - durch die Beschichtung wird weniger Reibung an den Lippen des verzehrenden bzw. konsumierenden Menschen erzeugt und auch ein kleben des Trinkhalms oder des Hilfsmittels kann somit vermieden werden.

Die Zugabe von Geruchsneutralisatoren führt zur Geruchsneutralisation und verhindert daher eventuell auftretende störende Gerüche.

Die Zugabe eines oder mehrerer "anti-sick" Zusätze, auch "Anti-sticking-Agents" oder "anti-tack" Zusätze, bzw. "Anti-Tack-Agents" zur Beschichtungsemulsion verhindert das Aneinanderkleben der erfindungsgemäßen Hilfsmittel zum Essen oder zum Trinken, bzw. der medizinischen Bedarfsmittel, wenn diese übereinanderliegend gelagert werden. Ferner verhindert die Zugabe eines oder mehrerer anti-stick Zusätze, dass die Beschichtung eines Hilfsmittels zum Essen oder zum Trinken durch Reibung, wie sie etwa beim Durchstechen von Plastikfolien entsteht, abgerieben wird.

### Abbildungen

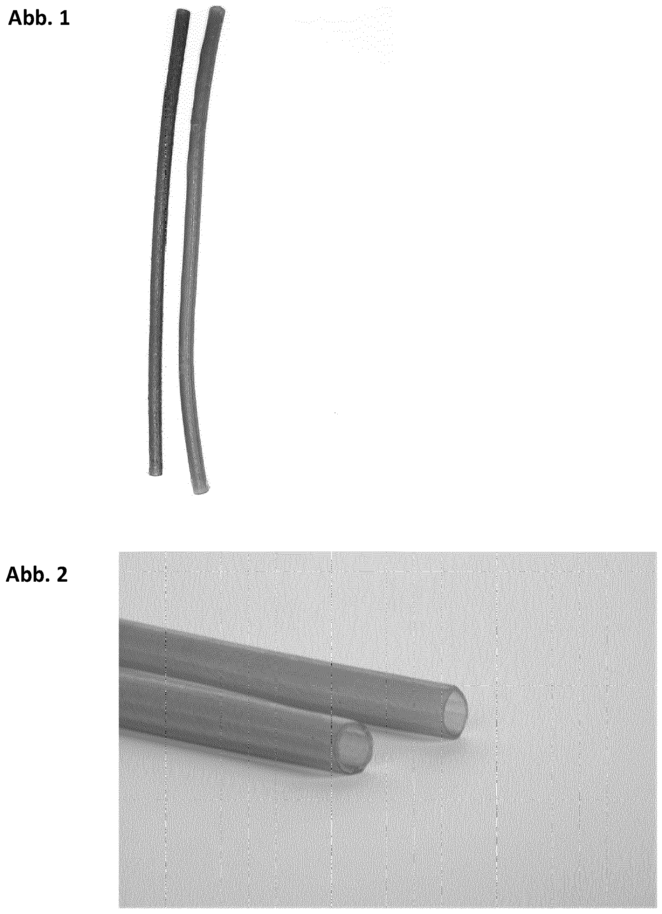

**Abbildung 1** zeigt erfindungsgemäße Trinkhalme. Diese sind teils mit verschiedenen Hilfsstoffen (Farbstoffen) eingefärbt.

**Abbildung 2** zeigt vergrößerte erfindungsgemäße Trinkhalme. Diese sind nicht eingefärbt.

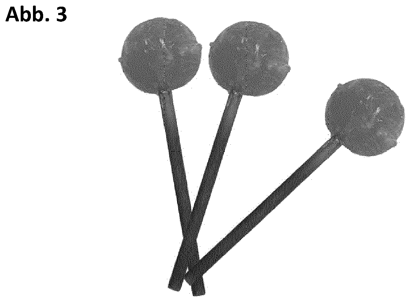

**Abbildung 3** zeigt Lollis mit erfindungsgemäßen Lollistielen / Lollisticks. Die Lollisticks sind eingefärbt.

### Ausführliche Beschreibung

### Definitionen

**"Hilfsmittel zum Essen oder zum Trinken"** umfassen Trinkhalme, Trinkbecher, Besteck, Lollisticks / Lollistiele, Sushi-Stäbchen und Eisstiele.

**"Trinkhalm"** und **"Strohhalm"** werden in der vorliegenden Anmeldung synonym verwendet.

**"Besteck"** ist ein Sammelbegriff für verschiedene Werkzeuge, mit denen Lebensmittel serviert, zerkleinert und gegessen werden. Besteck umfasst dementsprechend Messer, Gabeln, Pommesgabeln, Esslöffel, Teelöffel, Kuchengabeln, Eislöffel, sowie Abwandlungen hiervon.

**"medizinische Bedarfsartikel"** umfassen Wattestäbchen, Ohrenstäbchen, Mundspatel bzw. Mundspachtel, Einmalzahnbürsten, Einwegzungenreiniger.

**"Pflanzliche Stärke"** bezieht sich auf jegliche aus Pflanzenmaterial gewonnene Stärke. Die Stärke kann dabei etwa aus Wurzeln, Rüben, Knollen, Rhizomen, Sprossachsen, Blättern, Früchten oder Samen gewonnen werden. Beispielhaftes pflanzliche Stärken sind Weizenstärke, Kartoffelstärke, Maisstärke oder Tapiokastärke; Stärke aus Maniok (*Manihot esculenta*), Knollenbohne (*Pachyrhizus tuberosus*)*,* Batate (*Ipomoeo batatas*), Yamswurzel (*Dioscorea spec.*)*,* Knollen-Platterbse (*Lathyrus tuberosus*)*,* Arakacha (*Arracacia xanthorrhiza*), Knolligem Sauerklee (*Oxalis tuberosa*), Knolliger Kapuzinerkresse (*Tropaeolum tuberosum*)*,* Ulluco (*Ullucus tuberosus*)*,* Ostindischer Pfeilwurz (*Tacca leontopetaloides*), Pfeilwurz (*Maranta spec.*)*,* Achira (*Canna indica*), Taro (*Colocasia esculenta*), Tannia (*Xanthosoma sagittifolium*), Weißer Seerose (*Nymphaea alba*), Gelber Teichrose (*Nuphar lutea*) oder Chayote (*Sechium edule*)*.*

**"Faserstoff"** bezeichnet aus Fasern gewonnenes Material, das für die Herstellung von Papier und Pappe verwendet wird. Faserstoffe bestehen zum einem Großteil aus Cellulose. Faserstoffe sind beispielsweise Zellstoff und Holzstoff.

**"Zellstoff"** bezeichnet die beim chemischen Aufschluss von Pflanzen, v.a. Holz, entstehende faserige Masse. Sie besteht zu einem Großteil aus Cellulose. Für die Trinkhalme der vorliegenden Erfindung wird bevorzugt Zellstoff mit kurzen Fasern verwendet. Eine besonders bevorzugte Faserlänge ist 0,8 mm bis 1,1 mm. Der Zellstoff der vorliegenden Erfindung kann von vielerlei Pflanzen stammen, etwa von Nadelbäumen, Laubbäumen oder Bambus. In besonders bevorzugten Ausführungsformen ist der Zellstoff zumindest teilweise aus Baobab-Pflanzenmaterial hergestellt. In einer anderen besonders bevorzugten Ausführungsform ist der Zellstoff zumindest teilweise aus Bambus-Pflanzenmaterial hergestellt.

**"Holzstoff"** bezeichnet die bei mechanischem Aufschluss von Pflanzen, v.a. Holz, entstehende faserige Masse. Holzstoff enthält, anders als Zellstoff für höherwertige Papiere, große Anteile an Lignin. Für die Hilfsmittel zum Essen oder zum Trinken der vorliegenden Erfindung wird bevorzugt Holzstoff mit kurzen Fasern verwendet. Eine besonders bevorzugte Faserlänge ist 0,8 mm bis 1,1 mm. In manchen Ausführungsformen der vorliegenden Erfindung kommt eine Kombination aus Holzstoff und Zellstoff zum Einsatz.

**"Pflanzliche** Dickungsmittel" und **"pflanzliche Geliermittel"** sind Dickungs- und Geliermittel aus pflanzlichen oder bakteriellen Ressourcen. Bevorzugt sind Dickungs- und Geliermittel aus pflanzlichen Ressourcen. Sie bewirken die Gelierung von Flüssigkeit. Beispielhafte pflanzliche Dickungs- oder Geliermittel sind Agar-Agar, Pektin, Carrageen, Alginate, Johannisbrotkernmehl, Guarkernmehl, Sago, Xanthan, Gummi Arabicum, Reismehl, Hartweizenmehl oder Hartweizengrieß.

**"Guarkernmehl"** (als Lebensmittelzusatzstoff E412) ist ein pflanzliches Dickungs- oder Geliermittel. Es wird aus gemahlenen Samen der Guarpflanze gewonnen. Besonders hervorzuheben ist, dass Guarkernmehl die Wirkung anderer pflanzlicher Verdickungs- oder Geliermittel erheblich verstärkt und daher gerne mit anderen pflanzlichen Verdickungs- oder Geliermitteln eingesetzt wird.

**"Xanthan"** (als Lebensmittelzusatzstoff E415, auch als "Xanthan" bezeichnet), ist ein bakterielles Dickungs- und Geliermittel. Es wird durch Bakterien der Gattung *Xanthomonas* aus zuckerhaltigen Substraten hergestellt. Bevorzugterweise stammt das Xanthan der vorliegenden Erfindung von Bakterien der Spezies *Xanthomonas campestris.*

**"Agar-Agar"** bezeichnet ein aus Algen gewonnenes Galactose-Polymer. Agar-Agar wird aus den Zellwänden von Algen, insbesondere von Rotalgen gewonnen.

**"Pektin"** (als Lebensmittelzusatzstoff E440a oder E440b) bezeichnet pflanzliche Polysaccharide, die im Wesentlichen α-1-4-glycosidisch verknüpfte Galacturonsäuren umfassen. Sie können zum Beispiel aus Schalen von Äpfeln, Zitronen und anderen Früchten gewonnen werden. Pektin ist ein pflanzliches Dickungs- oder Geliermittel und sorgt so dafür, dass Flüssigkeiten gelieren.

**"Carrageen"** (als Lebensmittelzusatzstoff E407) ist ein pflanzliches Dickungs- oder Geliermittel, das aus verschiedenen Rotalgenarten gewonnen wird.

**"Alginat"** ist ein pflanzliches Dickungs- oder Geliermittel und besteht aus Salzen der Alginsäure. Es kann aus getrockneten und gemahlenen Braunalgen extrahiert werden. Je nach Salz ist es unter den E-Nummern E401, E402, E403, E404 und E405 bekannt.

**"Johannisbrotkernmehl"** (als Lebensmittelzusatzstoff E410) ist ein pflanzliches Dickungs- oder Geliermittel, das aus Johannisbrotbaumsamen durch mahlen gewonnen wird. Das erhaltene Mehl ist weiß und geschmacksneutral.

**"Sago"** ist ein pflanzliches Dickungs- oder Geliermittel. Sago wird aus dem stärkereichen Mark verschiedener Pflanzenarten wie der Sagopalme, Maniok, oder Kartoffeln gewonnen. Häufig wird es als Granulat in Form kleiner Kügelchen angeboten. Sago quillt in heißer Flüssigkeit etwa um das Dreifache auf und wirkt beim Abkühlen stark bindend. Damit die Flüssigkeit nicht breiig wird, wird Sago nur so lange gekocht oder eingeweicht, bis die Kügelchen weich sind, aber noch ihre Form behalten.

**"Gummi** arabicum" (als Lebensmittelzusatzstoff E414) ist ein pflanzliches Dickungs- und Geliermittel, das aus dem harzigen Pflanzensaft von in Afrika beheimateten Akazienarten, wie beispielsweise *Acacia senegal*, gewonnen wird. Gummi arabicum kann sowohl in Pulver- als auch in Gummiform vorliegen.

**"Reismehl"** ist ein pflanzliches Dickungs- und Geliermittel, das durch die Entspelzung von Reiskörnern und anschließendes feines Mahlen entsteht. Je nach Verwendung von poliertem oder unpoliertem (braunen) Reiskörnern, erhält man weißes oder braunes Reismehl.

**"Hartweizenmehl"** ist ein pflanzliches Dickungs- und Geliermittel, das aus dem Hartweizen (*Triticum durum*) gewonnen wird, der auch unter Durum, Durumweizen oder Glasweizen bekannt ist. Die Hartweizenkörner werden geschält und anschließend mehrmals gemahlen, sodass Hartweizenmehl entsteht.

**"Hartweizengrieß"** ist ein pflanzliches Verdickungsmittel, das aus dem Hartweizen (*Triticum durum*) gewonnen wird, der auch unter Durum, Durumweizen oder Glasweizen bekannt ist. Wie bei der Hartweizenmehl-Herstellung werden die Hartweizenkörner geschält und anschließend gemahlen. Allerdings werden die Hartweißenkörner weniger häufig gemahlen als bei der Herstellung von Hartweizenmehl, so dass Hartweißengrieß eine gröbere Partikelstruktur aufweist.

**"Wachs"** bezeichnet jegliches in der Natur vorkommendes Wachs, wie etwa Carnaubawachs, Bienenwachs oder Sojawachs. Bevorzugt ist das Wachs in der vorliegenden Erfindung Carnaubawachs. Dem Wachs der vorliegenden Erfindung wurde vorzugsweise Biopolymere wie Kautschuk hinzugefügt. **"Carnaubawachs"** wird aus dem Blatt der Carnaubapalme (*Copernicia prunifera*) gewonnen. Die Blätter werden geerntet, durch Trocknen und mechanisches Einwirkung wird das Wachs abgetrennt. Das Wachs kommt in diversen Industrien zum Einsatz, wie in der Lebensmittel-, Kosmetik- und Pharma-Industrie. Unbehandeltes Carnaubawachs hat eine helle gelbliche, grünliche bis dunkelgraue Farbe, es ist von Luftbläschen durchsetzt, hart, spröde und wasserunlöslich. Carnaubawachs ist das härteste natürliche Wachs mit dem höchsten Schmelzpunkt von über 80 °C. Außerdem ist es verzehrbar, liegt also natürlich in Lebensmittelqualität vor und hat einen milden Geschmack. Dies ist bei Trinkhalmen und Besteck, das Carnaubawachs umfasst von Vorteil, da die Trinkhalme und Besteck so keinen Eigengeschmack aufweisen und auf natürliche Art und Weise ein Produkt in Lebensmittelqualität darstellen. Außerdem gestaltet die Zugabe von Carnaubawachs zu den anderen Stoffen wie pflanzliche Stärke, Agar-Agar und Zellstoff den Verarbeitungsprozess der Stoffe leichtgängiger, während die Festigkeit, Härte und Resistenz der zu erhaltenden Trinkhalme und Bestecke erhalten bleibt. In der vorliegenden Erfindung kommt Carnaubawachs bevorzugt in Pulverform zur Anwendung. Dem vorstehenden Wachs werden in besonderen Ausführungsformen Biopolymere wie Kautschuk beigefügt. **"Sojawachs"** wird aus reifen Sojabohnen (*Glycine max*) gewonnen. Die Sojabohnen werden geerntet und zunächst wird Sojaöl gewonnen. Dieses wird hydriert, unter Druck von ca. 200 bar und Temperaturen von etwa 140°C - 225 °C in der Anwesenheit eines metallischen Katalysators erhält man Sojawachs. Das Wachs kommt in diversen Industrien zum Einsatz, wie in der Kosmetik- und Kerzen-Industrie. Unbehandeltes Sojawachs hat eine sehr helle, cremeweiße, manchmal gelbliche Farbe, ist hart, spröde und wasserunlöslich. Sojawachs hat einen Schmelzpunkt von etwa 50 °C. Außerdem ist es verzehrbar und hat einen milden Geschmack. Dies ist bei Trinkhalmen und Besteck von Vorteil, da die Trinkhalme und Besteck so keinen Eigengeschmack aufweisen und ohne weiteren Aufarbeitungsschritt ein Produkt in Lebensmittelqualität darstellen. Außerdem gestaltet die Zugabe von Sojawachs zu den anderen Stoffen wie pflanzliche Stärke, Agar-Agar und Zellstoff den Verarbeitungsprozess der Stoffe leichtgängiger, während die Festigkeit, Härte und Resistenz der zu erhaltenden Trinkhalme und Bestecke erhalten bleiben. In der vorliegenden Erfindung kommt Sojawachs bevorzugt in Pulverform zur Anwendung. Dem vorstehenden Wachs werden in besonderen Ausführungsformen Biopolymere wie Kautschuk beigefügt. **"Rapswachs"** wird aus den Samen vom Raps (*Brassica napus*) oder auch von dem nahen Verwandten Ölrübsen (*Brassica rapa* subsp. *oleifera*) gewonnen. Die Samen werden geerntet und zunächst wird Rapsöl gewonnen. Durch die Härtung von Rapsöl wird Rapswachs gewonnen. Der Schmelzpunkt von Rapswachs liegt bei etwa zwischen 57°C und 61°C. Auch Rapswachs ist verzehrbar. **"Carnaubawachsemulsion"** beschreibt eine Emulsion basierend auf Carnaubawachs umfassed 30-60% (Carnaubawachs-)Feststoffgehalt und 70-40% Wasser und optional ein geringe Menge an Ammoniak (0,03%), bevorzugt enthält die Carnaubawachsemulsion 50% Carnaubawachs und 50% Wasser, optional 0,03% Ammoniak. Optional umfasstes Ammoniak reduziert ein Absetzen der Feststoffteile der Emulsion.

**"Biopolymer"** ist ein Polymer, das auf nachwachsenden Rohstoffen basiert und (biologisch) abbaubar ist. Ein beispielhaftes Biopolymer ist Kautschuk, auch Gummi eleasticum oder Resina elastica genannt. Kautschuk bietet eine höhere Resistenz gegen Wasser als vergleichbare Resistenz. Daher ist Kautschuk selbst oder Mischungen davon besonders vorteilhaft. Kautschuk wird in Form von Naturlatex verwendet, alternativ in Form einer Emulsion basierend auf Kautschuk und Wasser mit einem Feststoffgehalt von 40-60%, bevorzugt 50-65%, am bevorzugtesten 60% Kautschuk. Auch bestimmte erdölbasierte Polymere sind biologisch abbaubar und daher Biopolymere. Erfindungsmäße erdölbasiere Polymere umfassen Polyvinylalkohol (PVA), Polybutylenadipat-terephthalat (PBAT), Polybutylensuccinat (PBS), Polycaprolactone (PCL) und Polyglycolid (PGA). Die Biopolymere der vorliegenden Erfindung können auch modifiziert werden und sind somit **"biobasierte Polymere".** Diese umfassen Polylactid (PLA), Polyhydroxyalkanoate (PHA), Polyhydroxybutyrat (PHB), Stoffe auf Ligninbasis wie Thermoplaste.

**"Emulsion"** bezeichnet jegliches verteilte Gemisch mehrerer Bestandteile, wie beispielsweise einem oder mehrere Feststoffe und einem Lösungsmittel wie Wasser. Eine Emulsion kann eine Wachsemulsion, eine Carnaubawachemulsion, oder Kautschukemulsion sein. Eine erfindungsgemäße Emulsion umfasst beispielsweise Carnaubawachs, Paraffin und Wasser. Eine weitere erfindungsgemäße Emulsion umfasst Carnaubawachs, Kautschuk und Wasser. Auch Naturlatex ist eine Emulsion aus Kautschuk und Wasser.

**"Öl"** bezeichnet jegliches pflanzliche, mineralische oder tierische Öl. Bevorzugt ist das Öl in der vorliegenden Erfindung ein pflanzliches Öl. Besonders bevorzugt sind Nussöl und Sonnenblumenöl.

**"Kompostierbar"** bezeichnet die Eigenschaft eines Materials, nach 6 Monaten unter definierten aeroben Bedingungen zu 90% abgebaut zu werden. Die Kompostierbarkeit eines Materials kann nach DIN EN 13432, Version 2000-12, bestimmt werden. Kompostierbarkeit betrifft hierin sowohl die industrielle Kompostierung als auch die nicht-industrielle Kompostierung durch rein biologische Zersetzung bzw. Verrottung (Heimkompostierung). So sind die erfindungsgemäßen Hilfsmittel und medizinischen Bedarfsartikel sowohl mittels Inventivrotte als auch mittels Trockenvergärung innerhalb von 4-8 Wochen, bevorzugt innerhalb 4 Wochen kompistierbar, sodass sich die Hilfsmittel und Bedarfsartikel innerhalb dieser Zeit vollständig aufgelöst haben.

**"Biologisch abbaubar"** bezeichnet die Charakteristik eines Prozesses, einen organischen Stoff biologisch, das heißt durch Lebewesen oder ihre Enzyme, vollständig abzubauen. Dabei findet der Abbau des Stoffes unter aeroben Bedingungen in höchstens 10 Jahren, bevorzugt in 5 Jahren, noch bevorzugter in 1 Jahr statt.

**"Recycelbar"** heißt, dass das Material nach seiner Verwendung (etwa als Trinkhalm) durch einen Aufarbeitungsprozess (Recycling) als Material zur Herstellung eines neuen, nicht zur Verbrennung gedachten Produkts verwendet werden kann. Dies steht im Gegensatz zu Materialien, die nach ihrer Verwendung entweder durch Verbrennung verwertet oder dauerhaft deponiert werden müssen. Recycelbare Materialien sind etwa Zellstoff, Papier, Pappe und Cellulosehydrat.

**"Magnesiumstearat"** Magnesiumstearat ist das Magnesiumsalz der Stearinsäure und gehört zu den Kalkseifen. Es wird aus Fetten und Ölen unter Spaltung ihrer Glyceride mittels Magnesium, Seifen und Glycerin gewonnen. Magnesiumstearat wird zum Beispiel in der pharmazeutischen Industrie als Hilfsmittel zur Tabletten- oder Granulatherstellung verwendet. Magnesiumstearat wird auch in einigen Süßigkeiten verwendet. Magnesiumstearat kann sowohl aus Fetten tierischer als auch pflanzlicher Herkunft hergestellt werden. Oft wird Soja-, Raps- oder Maiskeimöl verwendet. Die Substanz ist auch nützlich, weil sie schmierende Eigenschaften aufweist, die verhindern, dass Inhaltsstoffe während der Komprimierung von chemischen Pulvern zu festen Tabletten an den Herstellungsgeräten haften bleiben. Magnesiumstearat ist das am häufigsten verwendete Gleitmittel für Tabletten. Bei der Herstellung gepresster Bonbons wirkt Magnesiumstearat als Trennmittel und wird zum Binden von Zucker in Hartbonbons wie Minzen verwendet. Magnesiumstearat wird allgemein als Schmiermittel in einer Konzentration zwischen 0,25% und 5,0% in der Herstellung von Tabletten, Kapseln und anderen oralen Darreichungsformen verwendet. Aufgrund seiner langjährigen Verwendung als Hilfsstoff in der Pharma-, Lebensmittel- und Kosmetikindustrie ist die Sicherheit von Magnesiumstearat gut dokumentiert.

**"Calciumstearat"** Calciumstearat wird zur Herstellung sogenannter non-tox Stabilisatoren von Kunststoffen, bevorzugt in Verbindung mit Zinkstearat, aber auch Bariumstearat oder Magnesiumstearat verwendet. Weiterhin dient es als Gleitmittel in pharmazeutischen Produkten und als Schmierstoff (Staufferfett) in der Papier- und Metall-verarbeitenden Industrie, als Hydrophobierungsmittel für Baustoffe sowie in der Sandaufbereitung. Technisches Calciumstearat wird durch Reaktion von Calciumchlorid mit dem Natriumsalz der Stearinsäure (meist verunreinigt mit dem Natriumsalz der Palmitinsäure) und anschließendes Auswaschen von Natriumchlorid gewonnen. Es ist ein Imprägniermittel für Textilien. In Kunststoffen kann es als Säurefänger oder Neutralisator in Konzentrationen von bis zu 1000 ppm, als Schmiermittel und als Trennmittel wirken. Es kann in plastischen Farbmittelkonzentraten verwendet werden, um die Pigmentbenetzung zu verbessern. In Hart-PVC kann es die Verschmelzung beschleunigen, den Fluss verbessern und das Anschwellen des Stempels verringern. Zu den Anwendungen in der Körperpflege- und Pharmaindustrie gehören Tablettenformtrennmittel, Antihaftmittel und Geliermittel. Calciumstearat ist Bestandteil einiger Arten von Entschäumern. Außerdem ist Calciumstearat ein Antibackmittel, welches die feie Beweglichkeit von Feststoffen ermöglicht und so das Zusammenbacken pulverförmiger Bestandteile verhindert. Bei der Papierherstellung wird Calciumstearat als Schmiermittel verwendet, um einen guten Glanz zu erzielen, und um Staubbildung und Faltenrisse bei der Papier- und Kartonherstellung zu verhindern. Eine Zugabe von etwa 0,1 bis 10% ist möglich.

**"Zinkstearat"** ist ein weißes Pulver mit einem Schmelzpunkt von 130°C, einem Flammpunkt von 277°C und einer Selbstentzündungstemperatur von 420°C. Die Molekülmasse beträgt 632,3 g/mol. Zinkstearat ist nicht wasserlöslich. Zinkstearat wird als Stabilisator für Emulsionen verwendet. In der Kartenmagie verwendet man Zinkstearat als Kartenpuder, welches die Gleitfähigkeit von Spielkarten verbessert. Zinkstearat wird auch als Schmierstoff bei der Kunststoffverarbeitung verwendet. Es verhindert ein Festsitzen von Polyamidteilen untereinander und ist ein Hilfsmittel bei Plastifizierproblemen. Wenn die Plastifizierschnecke das Material nicht richtig einzieht, kann dies durch Zugabe von ca. 0,2% Zinkstearat verbessert werden, besonders bei Polyamid 6.0. Wenn die Plastifizierschnecke das Material nicht richtig einzieht, kann dies durch Zugabe von ca. 0,2 % Zinkstearat verbessert werden.

**"Glycerin"** Glycerin ist der Trivialname und die gebräuchliche Bezeichnung von Propan-1,2,3-triol und ist ein Fettstoff der in der vorliegenden Erfindung als Schmiermittel und Feuchthaltemittel eingesetzt wird. Bevorzugt ist E 422. E 422 wird überwiegend aus pflanzlichen Fetten und Ölen gewonnen. Die Herstellung aus synthetischen oder tierischen Stoffen ist ebenfalls möglich. Bevorzugt ist "vegetarisches Glycerin" oder "veganes Glycerin", welches durch die Umesterung von pflanzlichen Ölen hergestellt wird. Als Lebensmittelzusatzstoff ist E 422 für alle Nahrungsmittel allgemein zugelassen und es existiert auch keine Höchstmengenbeschränkung für die Verwendung von Glycerin. In der vorliegenden Erfindung erhöht die Zugabe von Glycerin die Beweglichkeit des Materials, was vor allem bei den erfindungsgemäßen Trinkhalmen nützlich sein kann.

**"Hilfsstoffe"** sind Stoffe, die der vorliegenden Erfindung weitere vorteilhafte Eigenschaften verleihen, beispielsweise hinsichtlich der Form, Herstellbarkeit, Stabilität. Hilfsstoffe umfassen beispielsweise Antioxidantien, Bindemittel, Emulgatoren, Stabilisatoren, Farbstoffe, Duftstoffe bzw. Geruchsneutralisatoren und/oder Glanzmittel. Vorzugsweise sind diese Hilfsstoffe biologisch abbaubar. Geruchsneutralisatoren umfassen dabei Stoffe aus Vanille, Zitrone Lavendel oder Tanne. Duftstoffe oder Geruchsneutralisatoren sind vorzugsweise von der Beschichtung von erfindungsgemäßen Hilfs- und Bedarfsmitteln umfasst werden vorzugsweise in eine Beschichtungsemulsion im Verhältnis 1L Beschichtungsemulsion : 1 Teelöffel Geruchsneutralisator (Pulver oder Flüssigkeit) zugegeben.

**"Kautschuk"** betrifft Naturkautschuk vorliegend als Emulsion mit Wasser, sowie Naturlatex bzw. Naturlatexmilch, wie etwa Natur Latex Laguna der Firma Colok GmbH. Das Latex oder die Kautschukemulsion kann zusätzlich ein Trennmittel wie Struktol^{®} oder Ammoniak umfassen. Der bevorzugte Feststoffgehalt einer Kautschukemulsion liegt bei 60% Kautschuk. Naturlatex und Kautschukemulsion bezeichnen dieselbe Emulsion und sind austauschbar zu verstehen.

**"anti-sick Zusätze",** auch "Anti-sticking-Agents" oder "anti-tack" Zusätze, bzw. "Anti-Tack-Agents" verhindert das Aneinanderkleben erfindungsgemäßer Gegenstände bei direktem Kontakt mehrerer der erfindungsgemäßen Gegenstände bei der Aufbewahrung wie etwa in Aufbewahrungsbehältern. Die Termini sind dem Fachmann bekannt. Die genauen Zusammensetzungen solcher anti-stick bzw. anti-tack Zusätze sind Herstellerspezifisch. Anti-tack Zusätze kommen bevorzugt bei der Verwendung von Kautschukemulsion bzw. Latex zum Einsatz. Anti-stick Zusätze bzw. anti-tack Zusätze umfassen Öle, Glycerin, Lecithine, pflanzliche Fette und pflanzliche Stearate.

Jegliche Zusätze und Bestandteile der vorliegenden Erfindung sind für die Verwendung in Lebensmitteln zugelassen und basieren auf rein pflanzlicher Basis.

### Ausführungsformen der Erfindung

Die Hilfsmittel zum Essen oder zum Trinken, sowie die medizinischen Bedarfsartikel der vorliegenden Erfindung können unterschiedliche Zusammensetzungen haben. Immer umfassen sie pflanzliche Stärke und ein pflanzliches Dickungs- oder Geliermittel. Am bevorzugtesten ist Guarkernmehl als Dickungs- oder Geliermittel, gefolgt von Xanthan, Agar-Agar, Pektin, Carrageen, Alginat, Johannisbrotkernmehl, Sago, Gummi arabicum, Reismehl, Hartweizenmehl und Hartweizengries. Ferner können auch die aufgeführten Dickungs- oder Geliermittel in einer beliebigen Kombination von den aufgeführten Dickungs- oder Geliermitteln aufgeführt werden. Mehrere pflanzliche Dickungs- oder Geliermittel können z.B. in identischen Mengen (Verhältnis 1:1) eingesetzt werden. Die Mengen der mehreren Dickungs- und Geliermittel kann auch unterschiedlich sein. So kann in manchen Ausführungsformen das Verhältnis zweier Geliermittel zwischen 10:1 und 1:10, bevorzugt zwischen 10:1 und 1:5, noch bevorzugter zwischen 5:1 und 1:2 betragen. Beispielhafte Verhältnisse sind 5:1, 4:1, 3:1, 2:1 und 1:2.

In manchen Ausführungsformen umfassen die Hilfsmittel zum Essen oder zum Trinken und die medizinischen Bedarfsartikel auch Zellstoff und/oder Holzstoff, was die oben beschriebenen Vorteile mit sich bringt. Weiterhin können die Hilfsmittel zum Essen oder zum Trinken und die medizinischen Bedarfsartikel der vorliegenden Erfindung optional Wachs, bevorzugt Carnaubawachs oder Sojawachs, umfassen, was ebenfalls oben beschriebene Vorteile mit sich bringt. Die Hilfsmittel zum Essen oder zum Trinken und die medizinischen Bedarfsartikel der vorliegenden Erfindung können optional auch Öl, bevorzugt Nussöl, umfassen.

### Ausführungsformen umfassend pflanzliche Stärke und Agar-Agar

Die folgenden Ausführungsformen beziehen sich auf Hilfsmittel zum Essen oder zum Trinken bevorzugt auf Trinkhalme und Besteck umfassend pflanzliche Stärke und Agar-Agar.

Der Gehalt an pflanzlicher Stärke beträgt 30-50%, bevorzugt 35-50%, noch bevorzugter 35-45%. Ein beispielhafter Stärke-Gehalt ist 43%.

Die pflanzliche Stärke ist Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote oder eine Mischung davon.

Bevorzugt ist die pflanzliche Stärke Weizenstärke, Kartoffelstärke, Maisstärke, Tapiokastärke oder eine Mischung davon.

Der Gehalt an Agar-Agar beträgt 30-60%, bevorzugt 30-50%, noch bevorzugter 40-50%. Ein beispielhafter Agar-Agar-Gehalt ist 50%.

Der Gehalt an Zellstoff beträgt 0-10%, bevorzugt 1-10%, noch bevorzugter 2-10%. Ein beispielhafter Zellstoff-Gehalt ist 4%.

Der Gehalt an Holzstoff beträgt 0-10%, bevorzugt 1-10%, noch bevorzugter 2-10%. Ein beispielhafter Holzstoff-Gehalt ist 4%.

Zellstoff und Holzstoff können in Kombination eingesetzt werden. In diesem Fall beträgt der Gesamt-Gehalt an Zellstoff und Holzstoff 0-10%, bevorzugt 1-10%, noch bevorzugter 2-10%. Ein beispielhafter Gesamt-Gehalt ist 4%. Das Verhältnis von Zellstoff zu Holzstoff beträgt zwischen 10:1 und 1:10, bevorzugt zwischen 10:1 und 1:5, noch bevorzugter zwischen 5:1 und 1:2. Beispielhafte Verhältnisse sind 5:1, 4:1, 3:1, 2:1, 1:1 und 1:2. Der Zellstoff und der Holzstoff weisen bevorzugt Fasern mit einer Länge von 0,8-1,1 mm auf.

Der Gehalt an Wachs, bevorzugt Carnaubawachs, beträgt 0-10%, bevorzugt 1-10%, noch bevorzugter 1,5-7%. Ein beispielhafter Wachs-Gehalt ist 3%.

Der Gehalt an Öl, bevorzugt Nussöl, beträgt 0-1,5%, bevorzugt 0,1-1%, noch bevorzugter 0,2-0,7%. Ein beispielhafter Öl-Gehalt ist 0,5%.

In bevorzugten Ausführungsformen umfassen die Hilfsmittel zum Essen oder zum Trinken der vorliegenden Erfindung 30-50% pflanzliche Stärke, 30-50% Agar-Agar 1-10% Zellstoff und/oder 1-10% Carnaubawachs. In besonders bevorzugten Ausführungsformen umfassen die Hilfsmittel zum Essen oder zum Trinkender vorliegenden Erfindung 30-50% pflanzliche Stärke, 30-50% Agar-Agar, 1-10% Zellstoff und 1-10% Carnaubawachs.

In einer noch bevorzugteren Ausführungsform umfassen die Hilfsmittel zum Essen oder zum Trinken der vorliegenden Erfindung 43% pflanzliche Stärke, 50% Agar-Agar, 4% Zellstoff und 3% Carnaubawachs.

In einer ebenso bevorzugten Ausführungsform umfassen die medizinischen Bedarfsartikel der vorliegenden Erfindung 30-50% pflanzliche Stärke, 30-50% Agar-Agar 1-10% Zellstoff und/oder 1-10% Carnaubawachs. In besonders bevorzugten Ausführungsformen umfassen die medizinischen Bedarfsartikel der vorliegenden Erfindung 30-50% pflanzliche Stärke, 30-50% Agar-Agar, 1-10% Zellstoff und 1-10% Carnaubawachs.

In einer noch bevorzugteren Ausführungsform umfassen die medizinischen Bedarfsartikel der vorliegenden Erfindung 43% pflanzliche Stärke, 50% Agar-Agar, 4% Zellstoff und 3% Carnaubawachs.

In bevorzugten Ausführungsformen sind die Hilfsmittel zum Essen und zum Trinken ein Trinkhalm und/oder Besteck. Daher umfasst die vorliegende Erfindung auch Trinkhalme und Besteck umfassend 30-50% pflanzliche Stärke, 30-50% Agar-Agar 1-10% Zellstoff und/oder 1-10% Carnaubawachs. Besonders bevorzugt sind Trinkhalme und/oder Besteck umfassend 43% pflanzliche Stärke, 50% Agar-Agar, 4% Zellstoff und 3% Carnaubawachs.

### Ausführungsformen umfassend pflanzliche Stärke und pflanzliches Dickungs- oder Geliermittel, bevorzugt Guarkernmehl und Xanthan

Die folgenden Ausführungsformen beziehen sich auf Hilfsmittel zum Essen oder zum Trinken sowie medizinische Bedarfsartikel umfassend pflanzliche Stärke und pflanzliches Dickungs- oder Geliermittel, bevorzugt Guarkernmehl und Xanthan.

Der Gehalt an pflanzlicher Stärke beträgt 30-70%, bevorzugt 50-65%, noch bevorzugter 50-60%. Ein beispielhafter Stärke-Gehalt ist 50%.

Die pflanzliche Stärke ist Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote oder eine Mischung davon.

Bevorzugt ist die pflanzliche Stärke Weizenstärke, Kartoffelstärke, Maisstärke, Tapiokastärke oder eine Mischung davon.

Der Gehalt an pflanzlichen Dickungs- und Geliermittel beträgt 30-70%, bevorzugt 40-60%, noch bevorzugter 40-50%. Ein beispielhafter Dickungs- und Geliermittel-Gehalt ist 50%.

Das pflanzliche Dickungs- und Geliermittel ist Guarkernmehl, Xanthan, Agar-Agar, Pektin, Carrageen, Alginat, Johannisbrotkernmehl, Sago, Gummi arabicum, Reismehl, Hartweizenmehl oder Hartweizengries oder eine Mischung davon.

Bevorzugt ist das pflanzliche Dickungs- und Geliermittel eine Mischung aus Guarkernmehl und Xanthan.

Die Hilfsmittel zum Essen oder zum Trinken und die medizinischen Bedarfsartikel können optional Zellstoff umfassen. Der Gehalt an Zellstoff beträgt 0-10%, bevorzugt 1-10%, noch bevorzugter 2-10%. Ein beispielhafter Zellstoff-Gehalt ist 4%.

Die Hilfsmittel zum Essen oder zum Trinken und die medizinischen Bedarfsartikel können optional Holzstoff umfassen. Der Gehalt an Holzstoff beträgt 0-10%, bevorzugt 1-10%, noch bevorzugter 2-10%. Ein beispielhafter Holzstoff-Gehalt ist 4%.

Zellstoff und Holzstoff können in Kombination eingesetzt werden. In diesem Fall beträgt der Gesamt-Gehalt an Zellstoff und Holzstoff 0-10%, bevorzugt 1-10%, noch bevorzugter 2-10%. Ein beispielhafter Gesamt-Gehalt ist 4%. Das Verhältnis von Zellstoff zu Holzstoff beträgt zwischen 10:1 und 1:10, bevorzugt zwischen 10:1 und 1:5, noch bevorzugter zwischen 5:1 und 1:2. Beispielhafte Verhältnisse sind 5:1, 4:1, 3:1, 2:1, 1:1 und 1:2. Der Zellstoff und der Holzstoff weisen bevorzugt Fasern mit einer Länge von 0,8-1,1 mm auf.

Die Hilfsmittel zum Essen oder zum Trinken und die medizinischen Bedarfsartikel können optional Wachs umfassen. Der Gehalt an Wachs, bevorzugt Carnaubawachs oder Sojawachs, beträgt 0-10%, bevorzugt 1-10%, noch bevorzugter 1,5-7%. Ein beispielhafter Wachs-Gehalt ist 3%.

Die Hilfsmittel zum Essen oder zum Trinken und die medizinischen Bedarfsartikel können optional Öl umfassen. Der Gehalt an Öl, bevorzugt Nussöl, beträgt 0-4%, bevorzugt 0,1-3%. Ein beispielhafter Öl-Gehalt ist 2%.

Die Hilfsmittel zum Essen oder zum Trinken und die medizinischen Bedarfsartikel können optional Glycerin umfassen. Der Gehalt an Glycerin 0-10%, bevorzugt 0-3%. Ein beispielhafter Glycerin-Gehalt ist 2%.

In einer weiteren Ausführungsform umfassen die die Hilfsmittel zum Essen oder zum Trinken der vorliegenden Erfindung 40-50% pflanzliche Stärke, 35-45% Guarkernmehl und 1-5% Xanthan.

### Äußere Beschichtung

Alle genannten Ausführungsformen der erfindungsgemäßen Hilfsmittel zum Essen oder zum Trinken sowie medizinische Bedarfsartikel umfassend pflanzliche Stärke und pflanzliches Dickungs- oder Geliermittel, bevorzugt Guarkernmehl und Xanthan können ferner eine äußere Beschichtung umfassen. Die Beschichtung umfasst Carnaubawachs, und/oder Rapswachs, und/oder Paraffin, oder Mischungen davon. Dem Wachs können vorzugsweise Biopolymere beigefügt sein die auf nachwachsenden Rohstoffen basieren und (biologisch) abbaubar sind, wie Kautschuk bzw. Naturlatex, oder bestimmte erdölbasierte Polymere, die biologisch abbaubar sind wie beispielsweise Polyvinylalkohol (PVA), Polybutylenadipat-terephthalat (PBAT), Polybutylensuccinat (PBS), Polycaprolactone (PCL) und Polyglycolid (PGA). Alternativ können auch biobasierte Polymere wie Polylactid (PLA), Polyhydroxyalkanoate (PHA), Polyhydroxybutyrat (PHB), oder Stoffe auf Ligninbasis wie Thermoplaste von der Wachsschicht umfasst sein. Die Beschichtung kann je nach gewünschter Dicke 0-10%, vorzugsweise 0,5-2% der Zusammensetzung des Hilfsmittels oder des medizinischen Bedarfsartikels betreffen.

In einer bevorzugten Ausführungsform ist die Beschichtung eine Wachs-basierte Beschichtung, welche Wachs als Hauptbestandteil enthält (Wachschicht). Eine bevorzugte Wachemulsion besteht aus 30-50% Wachs, bevorzugt Carnaubawachs oder Rapswachs, 50-70% Wasser und optional Hilfsstoffe und/oder ein oder mehrere anti-stick Zusätze. Bevorzugt enthält die Wachs-basierte Emulsion 32,5% Carnaubawachs, 17,5% Paraffin und 50% Wasser oder aus 32,5% Carnaubawachs, 17,5% Naturlatex und 50% Wasser. Eine weitere bevorzugte Wachsemulsion besteht aus 50% Carnaubawachs und 50% Wasser.Besonders bevorzugt ist eine Naturlatex-basierte Beschichtung, welche Naturlatex als Hauptbestandteil enthält. Eine Naturlatex-basierte Emulsion besteht aus 50-95% Naturlatex 5-10% Wachs, bevorzugt Carnaubawachs oder Rapswachs, und optional Hilfsstoffe und/oder ein oder mehrere anti-stick Zusätze. Bevorzugt enthält die Naturlatex-basierte Emulsion 10% Carnaubawachs und 90% Kautschukemulsion, bestehend aus 60% Feststoff-Kautschuk und 40% Wasser, oder Naturlatexmilch, oder 5% Carnaubawachs, 45% Kautschuk und 50% Wasser, oder aus 5% Rapswachs, 45% Kautschuk und 50% Wasser oder aus 5% Carnaubawachs und 95% Naturlatex oder aus 5% Carnaubawachs, 2% Glycerin, 93% Naturlatex.

Zudem kann die äußere Beschichtung ein oder mehrere anti-stick Zusätze umfassen. Die Anwesenheit solcher Zusätze verhindert ein späteres Aneinanderkleben der einzelnen Hilfs- und Bedarfsmittel aneinander, wenn diese gestapelt oder aneinandergereiht oder nebeneinander gelagert und aufbewahrt werden. Anti-stick Zusätze der vorliegenden Erfindung sind Öle umfassend Rapsöl, Kokosöl und Sonnenblumenöl, bevorzugt Rapsöle. Das Öl wird zu 0-5%, bevorzugt zu 0,5-1%, am meisten bevorzugt zu 0,5% der Menge der Beschichtungsemulsion hinzugegeben. Ein weiterer anti-stick Zusatz ist Sonnenblumenlecithin oder Sojalecithin. Dies kann zusätzlich zu einem Öl hinzugegeben werden, damit sich das Öl mit der Wachsemulsion vermengt. Ein zusätzlicher anti-stick Zusatz ist ein Stearat, wie z.B. Magnesiumstearat pflanzlichen Ursprungs. Dieses kann in einer Menge von 0,25%-0,75%, bevorzugt von 0,3% zur Wachsemulsion dazu gegeben werden. Der optionale anti-stick Zusatz Glycerin kann entweder der Beschichtungsemulsion zugesetzt werden oder als eine Art zweite Beschichtung auf die ausgehärtete erste Beschichtung mittels Düsen aufgesprüht werden.

In einer Ausführungsform umfassen die Hilfsmittel zum Essen oder zum Trinken der vorliegenden Erfindung
40-50% pflanzliche Stärke,
35-50% pflanzliches Dickungs- oder Geliermittel
0-10% Zellstoff und/oder Holzstoff
0-10% Wachs, bevorzugt Carnaubawachs oder Sojawachs
0-3% Nussöl oder Sonnenblumenöl
0-2% Glycerin und
optional eine äußere Beschichtung.

In einer weiteren Ausführungsform umfassen die die Hilfsmittel zum Essen oder zum Trinken der vorliegenden Erfindung 65% pflanzliche Stärke, 30% Guarkernmehl und 5% Xanthan und optional eine äußere Beschichtung.

In einer weiteren Ausführungsform umfassen die die Hilfsmittel zum Essen oder zum Trinken der vorliegenden Erfindung 50% pflanzliche Stärke, 45% Guarkernmehl und 5% Xanthan und optional eine äußere Beschichtung.

In einer weiteren bevorzugten Ausführungsform umfassen die Hilfsmittel zum Essen oder zum Trinken der vorliegenden Erfindung
40-50% pflanzliche Stärke,
35-45% Guarkernmehl
1-5% Xanthan
0-10% Zellstoff und/oder Holzstoff
0-10% Wachs, bevorzugt Carnaubawachs oder Sojawachs
0-3% Nussöl oder Sonnenblumenöl
0-2% Glycerin
optional eine äußere Beschichtung.

In einer weiteren bevorzugteren Ausführungsform umfassen die Hilfsmittel zum Essen oder zum Trinken der vorliegenden Erfindung
40-50% pflanzliche Stärke,
35-45% Guarkernmehl
1-5% Xanthan
0-10% Zellstoff und/oder Holzstoff
0,5-10% Wachs, bevorzugt Carnaubawachs und/oder Rapswachs
0,1-3% Nussöl oder Sonnenblumenöl
0,1-2% Glycerin.
optional eine äußere Beschichtung.

In einer weiteren Ausführungsform umfassen die Hilfsmittel zum Essen oder zum Trinken der vorliegenden Erfindung
42% pflanzliche Stärke umfassend Weizenstärke, Maisstärke und/oder Tapiokastärke,
42% Dickungs- oder Geliermittel umfassend Guarkernmehl und Xanthan,
2% Wachs, bevorzugt Carnaubawachs und/oder Rapswachs,
3% Nussöl oder Sonnenblumenöl,
2% Glycerin und
9% Wachsschicht umfassend Carnaubawachs, und/oder Rapswachs, und/oder Paraffin. Besonders bevorzugt ist eine Wachsschicht umfassend Carnaubawachs, die zusätzlich Kautschuk enthält.

In einer weiteren Ausführungsform umfassen die Hilfsmittel zum Essen oder zum Trinken der vorliegenden Erfindung, bevorzugt Trinkhalme
50% Stärke
5% Xanthan
45% Guarkernmehl und
Farbstoff
sowie eine Beschichtung,
wobei die Beschichtungsemulsion aus 5% Carnaubawachs, 2% Glycerin, 43% Naturlatex und 50% Wasser besteht.

Diese Ausführungsform kann Rückstände von Glycerin und Öl die als flüssige Bestandteile während des Herstellungsverfahrens zugesetzt wurden umfassen.

Die genannten Ausführungsformen betreffen jegliches Hilfsmittel zum Essen oder zum Trinken. Die Ausführungsformen betreffen demnach Trinkhalme, Trinkbecher, Besteck, Lollitsticks/Lollistiels, Sushi-Stäbchen, Eisstiele etc.

Ferner betreffen die Ausführungsformen medizinische Bedarfsartikel wie Ohrenstäbchen, Wattestäbchen, Einmalzahnbürsten und Mundspatel.

In einer weiteren bevorzugten Ausführungsform umfassen die medizinische Bedarfsartikel der vorliegenden Erfindung
40-50% pflanzliche Stärke,
35-45% Guarkernmehl
1-5% Xanthan
0-10% Zellstoff und/oder Holzstoff
0-10% Wachs, bevorzugt Carnaubawachs und/oder Sojawachs
0-3% Nussöl oder Sonnenblumenöl
0-2% Glycerin
und optional eine äußere Beschichtung.

In einer weiteren bevorzugteren Ausführungsform umfassen die medizinische Bedarfsartikel der vorliegenden Erfindung
40-50% pflanzliche Stärke,
35-45% Guarkernmehl
1-5% Xanthan
0-10% Zellstoff und/oder Holzstoff
0,5-10% Wachs, bevorzugt Carnaubawachs und/oder Rapswachs
0,1-3% Nussöl oder Sonnenblumenöl
0,1-2% Glycerin
und optional eine äußere Beschichtung.

In einer weiteren Ausführungsform umfassen die medizinischen Bedarfsartikel der vorliegenden Erfindung
42% pflanzliche Stärke umfassend Weizenstärke, Maisstärke und/oder Tapiokastärke,
42% Dickungs- oder Geliermittel umfassend Guarkernmehl und Xanthan,
2% Wachs, bevorzugt Carnaubawachs und/oder Rapswachs,
3% Nussöl oder Sonnenblumenöl,
2% Glycerin und
9% Wachsschicht umfassend Carnaubawachs, und/oder Rapswachs, und/oder Paraffin. Besonders bevorzugt ist eine Wachsschicht umfassend Carnaubawachs, die zusätzlich Kautschuk enthält.

### Eigenschaften der Hilfsmittel und Bedarfsartikel

Die Hilfsmittel zum Essen oder zum Trinken sowie die medizinischen Bedarfsartikel der vorliegenden Erfindung sind biologisch abbaubar und/oder sogar kompostierbar. In manchen Ausführungsformen ist ein Hilfsmittel / Bedarfsartikel opak. In anderen Ausführungsformen ist ein Hilfsmittel / Bedarfsartikel halb-transparent. Die Farbe der Hilfsmittel zum Essen oder zum Trinken / medizinischen Bedarfsartikel kann mittels herkömmlicher Farbe, bevorzugt Lebensmittelfarbe, modifiziert werden.

**Hilfsmittel zum Trinken** - **Trinkhalm:** Die Wandstärke des Trinkhalms ist variabel. Die Wand des Trinkhalms kann eine Stärke zwischen 0,1 mm und 2 mm, bevorzugt zwischen 0,3 mm und 1,5 mm oder zwischen 0,5 mm und 1,3 mm haben. Beispielhafte Wandstärken sind hier 1 mm und 1,2 mm. Der Durchmesser Trinkhalms ist variabel. Der Durchmesser des Trinkhalms kann zwischen 1 mm und 3 cm, bevorzugt zwischen 3 mm und 1,5 cm oder zwischen 5 mm und 8 mm betragen. Beispielhafte Durchmesser sind hier 6 mm und 7 mm. Die Länge des Trinkhalms ist variabel. Die Länge des Trinkhalms kann zwischen 5 cm und 50 cm, bevorzugt zwischen 10 cm und 35 cm oder zwischen 15 cm und 30 cm betragen. Beispielhafte Längen sind hier 20 cm, 21 cm und 25 cm. In einer Ausführungsform beinhaltet der Trinkhalm zudem Konservierungsstoffe, um ein Verderben des biologisch abbaubaren Materials zu verhindern. Der Gehalt an Konservierungsstoffen beträgt 0-2%, bevorzugt 0,2-1%, noch bevorzugter 0,5%.

In einer Ausführungsform beinhaltet der Trinkhalm zudem Glycerin E 422 (veganes/vegetarisches Glycerin), um die Beweglichkeit bzw. die Biegeeigenschaft der erfindungsgemäßen Trinkhalme zu erhöhen.

Die Trinkhalme der vorliegenden Erfindung sind für eine Vielzahl von Verwendungszwecken geeignet. Sie eignen sich für eine Vielzahl von Getränken, wie z.B. Heißgetränke, Tee, Kaffee, Kaltgetränke, wie Saft, Saftschorlen, Limonaden, Wasser etc. oder alkoholische Getränke, wie z.B. Bier, Wein, Cocktails etc.

**Hilfsmittel zum Essen - Besteck:** Die Dicke des Bestecks ist variabel. Die Dicke des Bestecks kann zwischen 1 mm und 20 mm, bevorzugt zwischen 3 mm und 17 mm oder zwischen 5 mm und 15 mm liegen. Eine beispielhafte Dicke ist hier 10 mm.

In einer Ausführungsform beinhaltet das Besteck zudem Konservierungsstoffe, um ein Verderben des biologisch abbaubaren Materials zu verhindern. Der Gehalt an Konservierungsstoffen beträgt 0-2%, bevorzugt 0,2-1%, noch bevorzugter 0,5%.

Das Besteck der vorliegenden Erfindung ist für eine Vielzahl von Verwendungszwecken geeignet. Es eignet sich für eine Vielzahl von Speisen, wie z.B. warme Speisen, Suppen, Eintöpfe, Aufläufe, Fleisch- und Fischgerichte, Beilagen wie beispielsweise Kartoffeln, sowie für kalte Speisen wie Salate, Eis und Joghurt.

**Hilfsmittel zum Essen** - **Lollistiel/Lollistick:** Die Maße eines Lollistiels/Lollisticks der vorliegenden Erfindung sind variabel. Der Durchmesser kann zwischen 1mm und 4mm liegen. Die Wandstärke kann zwischen 0,5 und 1,5mm liegen. In manchen Ausführungen ist der Stick nicht hohl. Die Länge kann zwischen 10 und 15 cm liegen. In einer bevorzugten Ausführung hat ein Lollistiel/Lollistick einen Durchmesser von 4mm, eine Wandstärke von 0,5mm und eine Länge von 12 cm. Bei manchen Ausführungen wird in den Endabschnitt des Sticks, der die Zuckermasse trägt, ein Loch in die Wand gestochen. Dies dient zur Halterung der Zuckermasse.

**Medizinische Bedarfsartikel** - **Ohrenstäbchen:** Die Maße eines Ohrenstäbchens (oder Wattestäbchens) der vorliegenden Erfindung sind variabel. Der Durchmesser kann zwischen 1mm und 4mm liegen. Die Wandstärke kann zwischen 0,5 und 1,5mm liegen. In manchen Ausführungen ist das Stäbchen nicht hohl. Die Länge kann zwischen 10 und 15 cm liegen. In einer bevorzugten Ausführung hat ein Ohrenstäbchen einen Durchmesser von 4mm, eine Wandstärke von 0,5mm und eine Länge von 10 cm.

### Herstellungsverfahren

Die vorliegende Erfindung bietet auch Verfahren zur Herstellung von Hilfsmitteln zum Essen oder Trinken gemäß der vorliegenden Erfindung. Dabei umfasst das Verfahren die folgenden Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung umfassend pflanzliche Stärke und pflanzliches Dickungs- und Geliermittel und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu Hilfsmitteln zum Essen oder Trinken,
c. Aushärten des in Schritt b. geformten Hilfsmitteln zum Essen oder Trinken,
d. optional, Beschichten der in Schritt c. ausgehärteten Hilfsmittel zum Essen oder Trinken mit einer Emulsion umfassend Carnaubawachs, und/oder Rapswachs, und/oder Paraffin, oder Mischungen davon. Besonders bevorzugt ist eine Beschichtung umfassend Carnaubawachs, die zusätzlich Kautschuk enthält.

Die vorliegende Erfindung bietet auch Verfahren zur Herstellung von medizinischen Bedarfsartikeln gemäß der vorliegenden Erfindung. Dabei umfasst das Verfahren die folgenden Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung umfassend pflanzliche Stärke und pflanzliches Dickungs- und Geliermittel und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile.
b. Formen der Mischung aus Schritt a. zu medizinischen Bedarfsartikeln,
c. Aushärten der in Schritt b. geformten medizinischen Bedarfsartikel,
d. optional, Beschichten der in Schritt d. ausgehärteten medizinischen Bedarfsartikel mit einer Emulsion umfassend Carnaubawachs, und/oder Rapswachs, und/oder Paraffin, oder Mischungen davon.

### Schritt a:

In Schritt a. werden pflanzliche Stärke und Dickungs- oder Geliermittel mit Wasser vermischt. Dies geschieht mit einem Mixer, der die Mischung zu einer homogenen Masse verrührt. Die Mischung kann optional auch Zellstoff, Holzstoff, Wachs (bevorzugt Carnaubawachs oder Sojawachs) sowie Öl (bevorzugt Nussöl), Glycerin und/oder eine Wachsemulsion umfassen. Hierbei wird folgendermaßen vorgegangen:
**In Schritt a.1** werden zunächst die festen Bestandteile umfassend pflanzliche Stärke und Dickungs- und Geliermittel im trockenen, pulverförmigen Zustand gut miteinander vermischt. Dies ist wichtig, um einer Klümpchenbildung beim Mischen mit Wasser entgegenzuwirken.

Die pflanzliche Stärke in der Mischung in Schritt a.1 ist Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote oder eine Mischung davon.

Bevorzugt ist die pflanzliche Stärke in der Mischung in Schritt a.1 Weizenstärke, Kartoffelstärke, Maisstärke, Tapiokastärke oder eine Mischung davon.

Das pflanzliche Dickungs- und Geliermittel in der Mischung in Schritt a.1 ist Guarkernmehl, Xanthan, Agar-Agar, Pektin, Carrageen, Alginat, Johannisbrotkernmehl, Sago, Gummi arabicum, Reismehl, Hartweizenmehl oder Hartweizengries oder eine Mischung davon.

Bevorzugt ist das pflanzliche Dickungs- und Geliermittel eine Mischung aus Guarkernmehl und Xanthan.

In manchen Ausführungsformen umfassen die festen Bestandteile in Schritt a1. zudem Zellstoff und/oder Holzstoff. Der Zellstoff kann zumindest teilweise aus Baobab-Pflanzenmaterial oder Bambus-Pflanzenmaterial hergestellt.

Die Mengenanteile der Bestandteile an der Gesamtmenge der Mischung aus Schritt a.1 wird im Folgenden erläutert:
Die Menge an pflanzlicher Stärke beträgt 30-70%, bevorzugt 50-65%. Eine beispielhafte Stärke-Menge ist 50%.

Die Menge an pflanzlichem Dickungs- und Geliermittel beträgt 30-70%, bevorzugt 35-50%. Eine beispielhafte pflanzliche Dickungs- und Geliermittel-Menge ist 50%.

Die Menge an Zellstoff beträgt 0-10%, bevorzugt 1-10%, noch bevorzugter 2-10%. Eine beispielhafte Zellstoff-Menge ist 2%.

Die Menge an Holzstoff beträgt 0-10%, bevorzugt 1-10%, noch bevorzugter 2-10%. Eine beispielhafte Holzstoff-Menge ist 2%.

Zellstoff und Holzstoff können in Kombination eingesetzt werden. In diesem Fall beträgt die Gesamtmenge an Zellstoff und Holzstoff 0-10%, bevorzugt 1-10%, noch bevorzugter 2-10%. Eine beispielhafte Gesamt-Menge ist 2%. Das Verhältnis von Zellstoff zu Holzstoff beträgt zwischen 10:1 und 1:10, bevorzugt zwischen 10:1 und 1:5, noch bevorzugter zwischen 5:1 und 1:2. Beispielhafte Verhältnisse sind 5:1, 4:1, 3:1, 2:1, 1:1 und 1:2. Der Zellstoff und der Holzstoff weisen bevorzugt Fasern mit einer Länge von 0,8-1,1 mm auf.

In einer bevorzugten Ausführungsform umfasst die Mischung in Schritt a.1 der vorliegenden Erfindung
50-70% pflanzliche Stärke und
30-50% pflanzliches Dickungs- oder Geliermittel.

In einer noch bevorzugteren Ausführungsform enthält die Mischung in Schritt a.1 der vorliegenden Erfindung
65% pflanzliche Stärke,
30% Guarkernmehl und
5% Xanthan.

In einer weiteren bevorzugten Ausführungsform enthält die Mischung in Schritt a.1 der vorliegenden Erfindung
50% pflanzliche Stärke,
45% Guarkernmehl und
5% Xanthan.

Die Menge an pflanzlicher Stärke, Dickungs-oder Geliermittel und Zell-/Holzstoff summiert sich dabei auf eine Gesamtmenge der Mischung aus Schritt a.1 von 100%. Die weiteren Bestandteile der Mischung aus Schritt a (Gesamtmischung) wie zusätzliche feste Bestandteile wie Wachspulver oder die flüssigen Bestandteile aus Schritt a.2, werden am Nettogewicht der Mischung der festen Bestandteile aus Schritt a.1 berechnet und sind daher in % des Nettogewichts der Mischung aus Schritt a.1 angegeben.

**In Schritt a.1 können zusätzliche feste Bestandteileumfasst sein.** In manchen Ausführungsformen umfassen die festen Bestandteile in Schritt a1 zudem Wachspulver, bevorzugt Carnaubawachspulver, und/oder Rapswachspulver, und/ oder Sojawachspulver.

Die Menge an Wachspulver, bevorzugt Carnaubawachs oder Sojawachs, beträgt 0-10% des Nettogewichts der festen Bestandteile aus der Mischung in Schritt a.1, bevorzugt 1-10%, noch bevorzugter 1,5-4% des Nettogewichts der festen Bestandteile aus der Mischung in Schritt a.1. Eine beispielhafte Wachs-Menge ist 3% des Nettogewichts der festen Bestandteile aus der Mischung in Schritt a.1. Folgendes Beispiel soll die Berechnung für 3% Wachspulver erläutern: Bei einem Nettogewicht der Mischung aus pflanzlicher Stärke, pflanzlichem Dickungs-oder Geliermittel und optional Zell-/Holzstoff von 1000g werden zu dieser Mischung 30g Wachspulver dazugegeben. Danach werden die Bestandteile gut gemischt.

In einer weiteren Ausführungsform enthält die Mischung in Schritt a.1 der vorliegenden Erfindung
55% pflanzliche Stärke,
45% Guarkernmehl
5% Xanthan und
bezogen auf das Nettogewicht dieser Mischung
1% Carnaubawachspulver.

**In Schritt a.2** werden die flüssigen Bestandteile zur Mischung aus Schritt a.1 in einer Menge bezogen auf das Nettogewicht der Mischung aus a.1 in mL dazu gegeben und gut vermischt. Die flüssigen Bestandteile umfassen Wasser und optional weitere flüssige Bestandteile wie eine Wachsemulsion, Glycerin, und/oder Öl.

Eine bevorzugte Wachsemulsion besteht aus 30-50% Feststoff und 50-70% Wasser, bevorzugt aus 40% Feststoff und 60% Wasser. Diese Emulsion wird unter ständigem Rühren erhitzt bis sich der Feststoff verflüssigt und im Anschluss sofort zur Mischung aus a.1 gegeben. Folgendes Beispiel soll die Berechnung erläutern: Bei einer Zugabe an Wachsemulsion von 2% des Nettogewichts der Mischung aus a.1 beträgt das Volumen der Wachsemulsion bei einem Nettogewicht der Mischung aus a.1 von 1000g 20 mL. Das Wachs umfasst jedes lebensmittelechte Wachs wie Carnaubawachs, Sojawachs, Bienenwachs, Rapswachs, lebensmittelechte Paraffine oder eine Mischung davon. Bevorzugt wird eine Emulsion aus Carnaubawachs, und/oder Sojawachs, und/oder Rapswachs.

Optional wird zur Mischung aus a.1 Öl, bevorzugt Nussöl oder Sonnenblumenöl dazugegeben. Die Menge an zugegebenem Öl beträgt 1-5% des Nettogewichts der Mischung aus a.1 in mL, bevorzugt 2-4% des Nettogewichts der Mischung aus a.1 in mL, noch bevorzugter 4% des Nettogewichts der Mischung aus a.1 in mL. Folgendes Beispiel soll die Berechnung erläutern: Bei einem Nettogewicht der Mischung aus a.1 von 1000g werden zu dieser Mischung 20-40 mL Öl dazugegeben. Danach werden die Bestandteile erneut gut gemischt.

Optional wird zur Mischung aus a.1 Lebensmittelfarbe, bevorzugt in flüssiger Form zugegeben. Der Anteil der Lebensmittelfarbe kann dabei 1-50% des dazugegebenen Wassers ersetzen.

Die Temperatur der Masse beim Mischvorgang liegt zwischen 30 und 40°C, bevorzugt bei 35°C.

Schritt a.2 umfasst beispielhaft:
a.2 Zugabe von flüssigen Bestandteilen der Mischung zur Mischung aus Schritt a.1 umfassend bezogen auf das Nettogewicht der Mischung aus Schritt a.1
2% Carnaubawachsemulsion
4% Sonnenblumenöl
5% Glycerin und
40% Wasser
und Mischen dieser Bestandteile

**Tabelle 1** zeigt weitere bevorzugte Mischungen gemäß Schritt a, d.h. gemäß den Schritten a.1 und a.2.

**Tabelle 1**

| Mischung Schritt a umfassend: | **Feste Bestandteile** (Mischung Schritt a.1) | | | Feste Bestandteile (Angaben in % des Nettogewichts der Mischung des festen Bestandteile aus a.1, in g) | **Flüssige Bestandteile** (Angaben in % des Nettogewichts der Mischung der festen Bestandteile aus a.1, in mL) | | | |
|---|---|---|---|---|---|---|---|---|
| Mischung Schritt a | Pflanzliche Stärke (z.B. Weizen-, Mais-Kartoffel-, Tapiokastärke) | Pflanzliches Dickungs-und Geliermittel (z.B. Guarkernmehl, Xanthan, Agar-Agar) | Zellstoff/ Holzstoff | Wachs in Pulverform | Öl (z.B. Sonnenblumenöl, Nussöl) | Glycerin | Wachsemulsion | Wasser |
| 1 | 50% Maisstärke | 50 % Agar- Agar | | | 4% | 5% | | 40% |
| 2 | 70% | 30% | | | 4% | 10% | | 40% |
| 3 | 60% | 40% | | | 4% | 8% | | 40% |
| 4 | 55 % | 45 % | | | 4% | 6% | | 40% |
| 5 | 50% | 49 % | 1% | 2 % | 3% | 2% | | 40% |
| 6 | 47% | 51% | 2% | 1 % | 2% | 1% | | 40% |
| 7 | 50% | 50% | | 5 % | 0,5% | 15% | | 30% |
| 8 | 50 % | 50 % | | 8 % | 0,8% | 20% | | 30% |
| 9 | 50% | 50% | 3% | | 1% | 25% | | 30% |
| 10 | 38,5 % | 61,5 % | | 1,5 % | 5% | 5% | | 35% |
| 11 | 52 % | 48% | | | 7% | 4% | | 35% |
| 12 | 33 % | 60 % | 5% | 2 % | 6% | 3% | | 35% |
| 13 | 30% | 70% | | | 8% | 2% | | 45% |
| 14 | 50% Weizenstärke | 45% Guarkernmehl 5% Xanthan | | | 4% Sonnenblumenöl | 5% | 2% Carnabauwachs | 40% |
| 15 | 55% Maisstärke | 40% Guarkernmehl 5% Xanthan | | 1% Carnabauwachs | 3% Sonnenblumenöl | 5% | | 40% |
| 16 | 52% Maisstärke | 43% Guarkernmehl 5% Xanthan | | | 4% Sonnenblumenöl | 5% | 2% Carnabauwachs | 40% |
| 17 | 50% Tapiokastärke | 45% Guarkernmehl 5% Xanthan | | | 2% Nussöl | | 4% Rapswachs | 30% |
| 18 | 55% Weizenstärke | 40% Guarkernmehl 5% Xanthan | | 2% Sojawachs | 4% Sonnenblumenöl | 10% | | 40% |
| 19 | 65% | 30% Guarkernmehl 5% Xanthan | | | 4% | | | 35% |
| 20 | 50% | 45% Guarkernmehl 5% Xanthan | | | 4% | | | 40% |
| 21 | 50% Weizenstärke | 45% Guarkernmehl 5% Xanthan | | | 4% Sonnenblumenöl | | 2% Carnabauwachs | 40% |
| **Minimum** - **Maximum** | **30%-70%** | **30%-70%** | **0%-10%** | **0%-10%** | **0,5%-10%** | 1%-25% | **0%-10%** | **30-50%** |

### Schritt b:

Formen der Mischung aus Schritt a zu einem Hilfsmittel zum Essen oder Trinken oder zu einem medizinischen Bedarfsartikel.

In Schritt b. wird die Mischung aus Schritt a. zu einem Hilfsmittel wie etwa zu einem Trinkhalm geformt. Schritt b wird im Folgenden anhand des Beispiels Trinkhalm erläutert. Durch Änderung der Matrize kann jedoch jegliche Form hergestellt werden.

Die Mischung aus Schritt a wird über ein Auslass-Ventil der Mischvorrichtung in einen Trichter geleitet. Über einen "Screw Conveyor" wird die Mischung weiter transportiert. In einer "Single Screw Extruder"-Maschine, wird die Mischung mittels einer Schnecke (Plastifizierschnecke) zu einem Auslass transportiert und durch einen Auslass in ihre Form gepresst. Dadurch bildet sich die gewünschte Trinkhalm-Form. Die Plastifizierschnecke spitzt sich dabei zum Ende, an welchem sich der Auslass befindet zu, d.h. der Durchmesser der Schnecke wird vom Einlass bis zum Auslass kleiner wodurch der Druck auf die durchlaufende Mischung stets steigt je näher sie dem Auslass kommt. Die Plastifizierschnecke ist selbst in einem Rohr angeordnet welches von verschiedenen Heizbändern ummantelt ist. Zwischen den Heizbändern und dem Rohr ist zudem eine Wasserkühlung angeordnet. Diese gewährleistet keine Überhitzung der Schnecke. Die Heizbänder können unterschiedliche Temperaturen aufweisen. Von Einlass bis Auslass der Schnecke im Rohr sind aufeinanderfolgend Heizbänder angeordnet. Die Temperaturen dieser Heizbänder können 50°C, 65°C, 70°C und 50°C betragen. Durch solch eine Anordnung soll gewährleistet werden, dass die Temperatur des Materials ständig zwischen 50°C und 70°C beträgt. Die Temperatur der Masse bei diesem Schritt ist ausschlaggebend für die Festigkeit und Geschmeidigkeit des späteren Materials. Eine zu niedrige Temperatur bewirkt keine Aktivierung der Gelierungsreaktion was das Material des Trinkhalms spröde werden lässt. Eine zu hohe Temperatur führt jedoch dazu, dass das Material Verbrennungserscheinungen wie Verfärbungen aufweist, spröde und/oder brüchig wird. Eine zu hohe Temperatur kann auch zu Blasenbildung im Material führen. Beides ist unerwünscht.

Die zum Auslass zugespitzte, enger werdende Form der Plastifizierschnecke führt zu immer größer werdender Druckeinwirkung auf das sich durch die Schnecke bewegende Mischung welcher zudem zu einer Temperaturerhöhung führt. Das letzte Heizband ist weniger hoch temperiert, da die Wärme in der Mischung durch Druck und Reibung entsteht. Beste Ergebnisse konnten mit einer Schnecken-Geschwindigkeit von 30 Hz bis 50 Hz erzielt werden. Hierdurch wird bei der Formung der Hilfsmittel das Material der Mischung aus Schritt a sehr dicht zusammengepresst was zu einer hohen Materialdichte führt, die zur Bruchfestigkeit der Trinkhalme beisteuert. Durch diese Materialdichte wird der Trinkhalm sehr hart und bruchsicher. Ferner wird durch den hohen Druck auch das Wasser in der Mischung herausgepresst welches durch Düsen in Form von Wasserdampf abgelassen wird. Das verdichtete Material wird am Auslass der Plastifizierschnecke in ein trichterförmiges Sammelbecken gepresst. Dieses läuft zunächst mit dem Material voll. Im Anschluss wird durch den Druck der aus dem Auslass der Schnecke stets nachfließenden Masse die im Sammelbecken befindliche Masse durch einen weiteren Auslass im Sammelbecken gepresst. Der Auslass umfasst mehrere runde Formen, auch Matrizen genannt, welche zur Formgebung mehrerer Trinkhalme (bzw. anderen länglichen Hilfsmitteln wie Lollistiele oder Haltestiele eines Ohrenstäbchens) führen. Die so durch Extrusion entstehenden (Trinkhalm)Stränge werden auf einen Kamm gelegt und mittels eines Förderbands weiter transportiert. Die geformten Stränge sind heiß (ca. 66°C) und das noch im Material enthaltene Wasser verdampft. Die Stränge für Trinkhalme weisen dabei eine Wandstärke von 1 mm und einen Durchmesser von 7mm auf.

Dieses sogenannte "Pressverfahren" ist sehr zeitsparend, sodass Schritt b in einer Zeitspanne von 4-10 min, bevorzugt 5 min durchgeführt werden kann (von Zugabe der Mischung bis zum gepressten Trinkhalmstrang, welcher den Auslass des Sammelbeckens verlässt).

Dasselbe Verfahren wird zum Formen eines Lollistiels angewandt. Der Durchmesser der Auslässe ist dann geringer. Ein Lollistiel/Lollistick der vorliegenden Erfindung hat einen Durchmesser von 3 mm, eine Wandstärke von 1 mm und eine Länge von 12 cm.

Dasselbe Verfahren wird zum Formen eines Haltestabes für Ohrenstäbchen angewandt.

Formen weiterer Hilfsmittel zum Essen oder Trinken sowie von medizinischem Hilfsmaterial: Um zum Beispiel Besteck oder andere "flache" Hilfs- oder Bedarfsartikel zu erhalten, wird die Mischung nachdem sie mittels einer Plastifizierschnecke verdichtet worden ist über den Auslass herausgepresst. Der Auslass hat die Form eines nicht geschlossenen Kreises. Sobald die gepresste Masse auf der Oberfläche eines Förderbandes aufliegt, klappen sich die beiden "Kreisenden" zur Seite weg und es entsteht ein flaches Band der Masse. Dieses wird mittels Förderband zu einer Walzmaschine befördert wo es zu Teigplatte mit gewünschter Dicke gewalzt wird. Anschließend werden die gewünschten Hilfsmittel zum Essen oder Trinken wie Bestecke wie Gabel, Löffel und Messer, bzw. medizinische Hilfsmittel wie Mundspatel aus der gewalzten Teigplatte mit einem Zylinder ausgestanzt. Die zum Ausstechen verwendete Form erlaubt die Formgebung des jeweilig gewünschten Hilfsmittels durch pressen der flachen Teigplatte in die gewünschte zwei- oder dreidimensionale Form. Dadurch bildet sich die gewünschte Besteck-Form.

### Schritt c:

In Schritt c. werden die in Schritt b geformten Hilfsmittel gehärtet. Dies kann zum Beispiel durch Trocknung mittels Belüftung/Ventilation geschehen. Dabei werden die geformten Hilfsmittel in eine "Cooling und Cutter"-Maschine eingeführt (auch Kühlungstunnel genannt), wo sie durch Ventilatoren getrocknet und dadurch gehärtet werden. Die darauffolgende Härtung mittels Wärme in einem Heiztunnel bzw. Trocknungstunnel wird bei einem Temperaturbereich zwischen 25 und 100°C, bevorzugt bei 85°C durchgeführt. Hierbei wird Luft mit entsprechender Temperatur durch den Tunnel geblasen.

Das Verfahren zur Herstellung des Hilfsmittels kann optional weitere Schritte umfassen. Zum Beispiel, falls das Hilfsmittel ein Trinkhalm, Lollistiel/Lollistick, Haltestiel für Ohrenstäbchen oder ein ähnliches längliches Hilfsmittel ist, können die jeweiligen Stränge, die in Schritt b geformt wurden in der Länge zu einzelnen Trinkhalmen, Lollistielen/Lollisticks, Haltestielen für Ohrenstäbchen oder ähnlichen länglichen Hilfsmitteln gekürzt werden. Dies geschieht durch in der "Cooling und Cutter"-Maschine durch beispielsweise ein Messer

Das Verfahren zur Herstellung der Hilfsmittel kann zudem die Bestrahlung der Hilfsmittel mit UV-Licht beinhalten. Dies dient zur Desinfektion der Hilfs- und Bedarfsartikel.

In manchen Ausführungsformen wird das Hilfsmittel zudem bedruckt. Zum Bedrucken können alle dem Fachmann bekannten geeigneten Druckverfahren verwendet werden, z.B. Flexodruckverfahren oder Laserdruckverfahren. Dabei können etwa eine Druckmaschine von Guowei, GWR, oder Hangzhou Colon Machinery Co., Ltd., Modell CL-DC850, zum Einsatz kommen. Der Druck erfolgt bevorzugt mit ungiftigen Farben. In besonders bevorzugten Ausführungsformen wird Lebensmittelfarbe zum Drucken verwendet. Schritte b und c haben hierbei eine Gesamtdauer von unter 30 min, bevorzugt von 20 min. Das gesamte Herstellungsverfahren weist demnach eine Gesamtdauer von 25-35 min, bevorzugt von 25 min auf. Diese sehr kurze Gesamtdauer schlägt sich in einem geringen Energieverbrauch nieder, was das Verfahren wirtschaftlich macht.

Durch die Verfahren der vorliegenden Erfindung kann eine große Menge an Hilfsmittel zum Essen oder zum Trinken bzw. an medizinischen Hilfsmitteln in kurzer Zeit hergestellt werden. Der Output des Verfahrens zur Herstellung von Trinkhalmen kann z.B. bei mindestens 50 kg/h, bevorzugt bei mindestens 100 kg/h, noch bevorzugter bei mindestens 150 kg/h liegen.

Der Output des Verfahrens zur Herstellung von Besteck kann z.B. bei mindestens 50 kg/h, bevorzugt bei mindestens 100 kg/h, noch bevorzugter bei mindestens 150 kg/h liegen.

Zudem ist die hierfür aufzuwendende Energie aufgrund dieser kurzen Zeit im Gegensatz zu üblichen Trocknungsverfahren in z.B. einem Trocknungstunnel sehr gering.

### Schritt d (optional):

Um die Resistenz gegen Feuchtigkeit wie Wasser zu erhöhen, können die durch das vorliegende Verfahren hergestellten Hilfsmittel zum Essen oder Trinken und medizinischen Bedarfsartikel mit einer dünnen Beschichtung basierend auf Wachs oder Naturlatex überzogen werden. Ferner verlieht die erfindungsgemäße Beschichtung dem Trinkhalm beziehungsweise den Hilfsmitteln weitere vorteilhafte Eigenschaften - durch die Beschichtung wird weniger Reibung an den Lippen des verzehrenden bzw. konsumierenden Menschen erzeugt und auch ein Kleben des Trinkhalms oder des Hilfsmittels kann somit vermieden werden. Somit wird auch ein Aneinanderkleben einzelner Hilfsmittel wie Trinkhalme oder Bedarfsmittel wie Wattestäbchen bei der Lagerung bzw. Transport verhindert.

Das Wachs und/oder der Naturlatex liegt dafür in einer Emulsion vor und wird auf zum Beispiel die Trinkhalme bzw. das Besteck aufgebracht, z.B. aufgesprüht. Bevorzugtes Wachs ist hierbei Carnaubawachs. Eine bevorzugte Wachemulsion besteht aus 30-50% Wachs, bevorzugt Carnaubawachs oder Rapswachs, 50-70% Wasser und optional Hilfsstoffe und/oder ein oder mehrere anti-stick Zusätze. Bevorzugt enthält die Wachs-basierte Emulsion 32,5% Carnaubawachs, 17,5% Paraffin und 50% Wasser oder aus 32,5% Carnaubawachs, 17,5% Naturlatex und 50% Wasser. Eine weitere bevorzugte Wachsemulsion besteht aus 50% Carnaubawachs und 50% Wasser. Eine Naturlatex-basierte Emulsion besteht aus 50-95% Naturlatex 5-10% Wachs, bevorzugt Carnaubawachs oder Rapswachs, und optional Hilfsstoffe und/oder ein oder mehrere anti-stick Zusätze. Bevorzugt enthält die Naturlatex-basierte Emulsion 10% Carnaubawachs und 90% Kautschukemulsion, bestehend aus 60% Feststoff-Kautschuk und 40% Wasser, oder Naturlatexmilch, oder 5% Carnaubawachs, 45% Kautschuk und 50% Wasser, oder aus 5% Rapswachs, 45% Kautschuk und 50% Wasser oder aus 5% Carnaubawachs und 95% Naturlatex oder aus 5% Carnaubawachs, 2% Glycerin, 43% Naturlatex und 50% Wasser.

Bei allen Wachsemulsionen zur Beschichtung kann alternativ auch Sojawachs, Bienenwachs oder Paraffinwachs verwendet werden. Ferner können die Emulsionen auch einen oder mehrere Biopolymere und /oder Hilfsstoffe umfassen.

Bevorzugt umfasst die Emulsion zum Beschichten der Hilfs- und Bedarfsmittel ferner einen oder mehrere anti-stick Zusätze welche die Reibung und das Anhaften zwischen einzelnen Hilfs- und Bedarfsmitteln deutlich verringert und somit ein Aneinanderkleben bei der Lagerung in z.B. einem kleineren Container verhindert. Diese anti-stick Zusätze umfassen Öle wie Rapsöl, Kokosöl, Sonnenblumenöl, Lecithine wie Sonnenblumenlecithin oder Sojalecithin, sowie pflanzliches Magnesiumstearat. Das eine oder mehrere Öle, bevorzugt Rapsöl, kann in einer Menge von bis zu 5%, bevorzugt 0,5%, zur Emulsion dazugegeben werden. Falls Öl als anti-stick Zusatz zur Emulsion dazu gegeben wird, ist eine weitere Zugabe von Sonnenblumenlecithin oder Sojalecithin vorteilhaft. Es hilft bei der Vermengung der Bestandteile der Emulsion. Ferner kann der Emulsion pflanzliches Magnesiumstearat in einer Menge von bis zu 3%, bevorzugt 0,25-0,75% hinzugefügt werden. Ferner kann die Emulsion Glycerin umfassen.

Einer Emulsion umfassend Glycerin kann das Glycerin entweder direkt der Emulsion beigefügt werden oder als eine zweite Beschichtung auf die erste Beschichtung basierend auf der Emulsion aufgesprüht werden. Dies dient als Schmierfilm und verhindert an ankleben.

Die Beschichtung mit der Emulsion kann auf unterschiedliche Arten und Weisen erfolgen. Die Beschichtung kann nur außen oder nur innen vorliegen oder sowohl außen und innen vorliegen.

In einer Ausführungsform werden die Hilfs- und Bedarfsartikel kurz in ein Becken mit Wachsemulsion gegeben und danach getrocknet.

In einer weiteren Ausführungsform werden die Hilfs- und Bedarfsartikel in eine Beschichtungsmaschine befördert, wo sie mittels Sprühdüsen mit einer Wachsemulsion beschichtet und anschließend getrocknet werden. Bei einer Auftragung mittels Sprühverfahren kann der Emulsion, optional Ethanol in einer Menge von bis zu 2%, bevorzugt 0,3% zugesetzt werden.

Eine aufgebrachte Beschichtung verleiht dem Hilfsmittel zudem eine glatte und glänzende Oberfläche.

Um die Reibung und die Anhaftung der einzelnen Hilfs- oder Bedarfsmittel bei der Lagerung zu verringern, kann das mit einer Emulsion beschichtete Hilfs- oder Bedarfsmittel alternativ zur Beimischung von ein oder mehreren anti-stick Zusätzen mit einer dünnen Schicht einer pulverförmigen Komponente wie Stearate oder Mehl bestäubt werden. Dazu werden die jeweiligen Hilfs- oder Bedarfsmittel, zum Beispiel Trinkhalme, in eine sogenannte "Dustbox" oder "Staubbox" gelegt, darin gewendet, herausgeholt und überschüssiges Pulver abgeklopft.

### Bevorzugte Ausführungsformen

In einer bevorzugten Ausführungsform umfasst das Verfahren für die Herstellung von Hilfsmitteln zum Essen oder zum Trinken und für die Herstellung von medizinischen Bedarfsartikeln folgende Schritte:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung enthaltend
      50% Weizenstärke
      45% Guarkernmehl
      5% Xanthan
      und Mischen dieser Bestandteile,
   a.2 Zugabe der flüssigen Bestandteile der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

In einer weiteren bevorzugten Ausführungsform umfasst das Verfahren für die Herstellung von Hilfsmittel zum Essen oder zum Trinken und für die Herstellung von medizinischen Bedarfsmitten folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      55% Maisstärke
      40% Guarkernmehl
      5% Xanthan und
      bezogen auf das Nettogewicht dieser Mischung
      1% Carnaubawachspulver
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      3% Sonnenblumenöl
      5% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

In einer bevorzugten Ausführungsform umfasst das Verfahren für die Herstellung von Hilfsmittel zum Essen oder zum Trinken und für die Herstellung von medizinischen Bedarfsartikeln folgende Schritte:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung enthaltend
      52% Maisstärke
      43% Guarkernmehl
      5% Xanthan
      und Mischen dieser Bestandteile,
   a.2 Zugabe der flüssigen Bestandteile der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

In einer bevorzugten Ausführungsform umfasst das Verfahren für die Herstellung von Hilfsmittel zum Essen oder zum Trinken und für die Herstellung von medizinischen Bedarfsartikels folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Tapiokastärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      4% Rapswachsemulsion
      2% Nussöl
      5% Glycerin und
      30% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

In einer bevorzugten Ausführungsform umfasst das Verfahren für die Herstellung von Hilfsmittel zum Essen oder zum Trinken und für die Herstellung von medizinischen Bedarfsartikeln folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      55% Weizenstärke
      40% Guarkernmehl
      5% Xanthan und
      bezogen auf das Nettogewicht dieser Mischung
      2% Sojawachspulver
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      4% Sonnenblumenöl
      10% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

In einer bevorzugten Ausführungsform umfasst das Verfahren für die Herstellung von Hilfsmittel zum Essen oder zum Trinken und für die Herstellung von medizinischen Bedarfsartikeln folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken oder einem medizinischen Bedarfsartikel,
c. Aushärten des in Schritt b. geformten Hilfsmittels zum Essen oder Trinken oder medizinischen Bedarfsartikels,
d. optional, Beschichten des in Schritt c. ausgehärteten Hilfsmittels zum Essen oder Trinken oder medizinischen Bedarfsartikels mit einer Wachs- oder Naturlatex-basierten Emulsion.

### Konkrete Ausführungsformen

Verfahren für die Herstellung von Trinkhalmen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Trinkhalm,
c. Aushärten des in Schritt b. geformten Trinkhalms,
d. Beschichten des in Schritt c. ausgehärteten Trinkhalms mit einer Naturlatexemulsion,
wobei die Carnaubawachsemulsion 50% Carnaubawachs und 50% Wasser enthält, und
wobei die Wachsemulsion zur Beschichtung aus 5% Carnaubawachs, 2% Glycerin, 43% Naturlatex und
50% Wasser besteht und optional einen oder mehrere anti-stick Zusätze enthält.

Verfahren für die Herstellung von Trinkhalmen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Trinkhalm,
c. Aushärten des in Schritt b. geformten Trinkhalms,
d. Beschichten des in Schritt c. ausgehärteten Trinkhalms mit einer Naturlatexemulsion,
wobei die Carnaubawachsemulsion 50% Carnaubawachs und 50% Wasser enthält, und
wobei die Wachsemulsion zur Beschichtung aus 5% Carnaubawachs, 95% Naturlatex oder
Kautschukemulsion und optional einen oder mehrere anti-stick Zusätze enthält.

Verfahren für die Herstellung von Trinkhalmen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      55% Weizenstärke
      40% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Sojawachspulver
      4% Sonnenblumenöl
      10% Glycerin
      2% Lebensmittelfarbe und
      40% Wasser
      und Mischen dieser Bestandteile
b. Formen der Mischung aus Schritt a. zu einem Trinkhalm,
c. Aushärten des in Schritt b. geformten Trinkhalms,
d. Beschichten des in Schritt c. ausgehärteten Trinkhalms mit einer Wachsemulsion,
wobei die Wachsemulsion zur Beschichtung aus 32,5% Carnaubawachs, 17,5% Paraffin und 50% Wasser
besteht und optional einen oder mehrere anti-stick Zusätze enthält.

Verfahren für die Herstellung von Besteck umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu Besteck,
c. Aushärten des in Schritt b. geformten Bestecks,
d. optional, Beschichten des in Schritt c. ausgehärteten Bestecks mit einer Wachsemulsion,
wobei die Carnaubawachsemulsion 50% Carnaubawachs und 50% Wasser enthält, und
wobei die Wachsemulsion zur Beschichtung aus 50% Carnaubawachs und 50% Wasser besteht und
optional einen oder mehrere anti-stick Zusätze enthält.

Verfahren für die Herstellung von Lollistielen/Lollisticks umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Lollistielen/Lollisticks,
c. Aushärten des in Schritt b. geformten Lollistiels/Lollisticks,
d. optional, Beschichten des in Schritt c. ausgehärteten Lollistiels/Lollisticks mit einer Wachsemulsion
wobei die Carnaubawachsemulsion 50% Carnaubawachs und 50% Wasser enthält, und
wobei die zur Beschichtung aus 32% Carnaubawachs, 17,5% Natur Latex und 50% Wasser besteht und
optional einen oder mehrere anti-stick Zusätze enthält.

Verfahren für die Herstellung von Ohrenstäbchen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Ohrenstäbchen,
c. Aushärten des in Schritt b. geformten Ohrenstäbchens,
d. optional, Beschichten des in Schritt c. ausgehärteten Ohrenstäbchens mit einer Wachsemulsion,
wobei die Carnaubawachsemulsion 50% Carnaubawachs und 50% Wasser enthält, und
wobei die Wachsemulsion zur Beschichtung aus 32% Carnaubawachs, 17,5% Natur Latex und 50% Wasser
besteht und optional einen oder mehrere anti-stick Zusätze enthält.

Verfahren für die Herstellung von Eisstielen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Nussöl
      4% Rapswachsemulsion
      30% Wasser
      und Mischen dieser Bestandteile
b. Formen der Mischung aus Schritt a. zu einem Ohrenstäbchen,
c. Aushärten des in Schritt b. geformten Ohrenstäbchens,
d. Beschichten des in Schritt c. ausgehärteten Ohrenstäbchens mit einer Wachsemulsion,
wobei die Rapswachsemulsion 50% Rapswachs und 50% Wasser enthält, und
wobei die Wachsemulsion zur Beschichtung aus 50% Sojawachs und 50% Wasser besteht und optional
einen oder mehrere anti-stick Zusätze enthält.

### Beispiele

Die vorliegende Erfindung wird durch die nachfolgenden, nicht einschränkenden Beispiele, detailliert

### Beispiel 1: Herstellung eines Trinkhalms

Das Ausgangsmaterial für den Trinkhalm bestand aus 50% Weizenstärke, 45% Guarkernmehl, 5% Xanthan. Zusätzlich bezogen auf das Nettogewicht plus 2% Carnaubawachsemulsion, 4% Sonnenblumenöl, 5% Glycerin und 40% Wasser. Diese Rohmaterialen, ausschließlich Wasser, Glycerin, Carnaubawachsemulsion und Sonnenblumenöl, wurden in einem Mixer verrührt. Dies geschieht solange bis sich die verschiedenen Bestandteile gut vermischt haben. Daraufhin wurde Wasser (+40% auf das Nettogewicht), Carnaubawachsemulsion (+2% auf das Netto- Gewicht), Glycerin (+5% auf das Nettogewicht) und Sonnenblumenöl (+4% auf das Nettogewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch sehr pulvrige Mischung wurde dann in einen "Screw Conveyor", welcher die Mischung weiter nach oben transportierte, gegeben. Anschließend wurde die Mischung in das Fütterungssystem der "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch 10 Auslasse (Matrizen) in ihre Form zu Strängen gepresst. Dadurch bildete sich die gewünschte vorläufige Trinkhalm-Form. Die geformten Trinkhalm-Stränge hatten eine Wandstärke von 1 mm und einen Durchmesser von 7 mm. Die Stränge wurden anschließend in einer "Cooling und Cutter"-Maschine" bearbeitet. Hierbei trockneten die Stränge, bei Durchlaufen des Förderbandes, wo an der Oberseite der Maschine angebrachte Ventilatoren das noch feuchte (35%) und warme (65°C) Material runter kühlten. Nach Durchlaufen des 10m langen Kühlungstunnels, liefen die Stränge in die Schneidemaschine, welche die Stränge in die gewünschte Länge von 20 cm mit einer von oben nach unten stoßenden Klinge zuschnitten. Schließlich wurden die zugeschnittenen Trinkhalme auf das Förderband eines Heiztunnels weiterbefördert, welcher die Trinkhalme bei circa 85 °C Heißluft und UV-Licht vollständig aushärtete und desinfizierte. Am Ende des Heiztunnels wurden die Trinkhalme in eine "Beschichtungsmaschine" befördert. Diese befördertet die Trinkhalme mittels eines Förderbandes mit Lamellen in ein Becken mit einer Emulsion bestehend aus: 5% Carnaubawachs, 2% Glycerin, 43% Naturlatex und 50% Wasser. Des Weiteren wurden Hilfsstoffe und ein oder mehrere anti-stick Zusätze hinzugegeben. Somit wurden die Trinkhalme sowohl innen, als auch außen beschichtet. Anschließend wurden die Trinkhalme auf einen weiteren Trocknungstunnel befördert und die Beschichtung härtete weiter aus. Zuletzt fielen die Trinkhalme in einen Karton und wurden anschließend nach Hygienevorschriften verpackt.

### Beispiel 2: Herstellung von Besteck (Messer)

Das Ausgangsmaterial für das Besteck (Messer) besteht aus 55% Maisstärke, 40% Guarkernmehl, 5% Xanthan. Zusätzlich bezogen auf das Nettogewicht plus 4% Rapswachs-Pulver, 4% Nuss-Öl, 35% Wasser und 4% blauer Lebensmittelfarbe. Diese Rohmaterialen, ausschließlich Wasser, Lebensmittelfarbe und Nuss-Öl, wurden in einem Mixer verrührt. Dies geschieht solange bis sich die verschiedenen Bestandteile gut vermischt haben. Daraufhin wurde Wasser (+40% auf das Nettogewicht), Lebensmittelfarbe (+4% auf das Nettogewicht) und Nuss Öl (+4% auf das Nettogewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch pulvrige Mischung wurde dann in einen "Screw Conveyor", welcher die Mischung weiter nach oben transportierte, gegeben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch einen Auslass (Matrize) in ihre Form eines fast schließenden Kreises gepresst. Die Masse öffnet sich und faltet sich aus. Dadurch bildete sich die gewünschte vorläufige Form. Mit einer Wandstärke von 2 mm. Die ca. 50 cm breite Teigplatte wird in den Ausstanz-Bereich befördert. Hierbei wird die Masse mit einer Walzmaschine zu der gewünschten Form (Messer) aus der flachen (2 mm) Teigplatte ausgestochen. Dadurch bildete sich die gewünschte Besteck-Form, in diesem Fall ein Messer. Das geformte Messer weist eine Dicke von 2 mm auf. Schließlich wurde das zugeschnittene Besteck (Messer) auf das Förderband eines Heiztunnels befördert, welcher das Besteck (Messer) bei circa 85°C Heißluft und UV-Licht vollständig aushärtete und desinfizierte. Am Ende des Heiztunnels wurden die Messer in eine Beschichtungsmaschine befördert. Diese beschichtete die Messer mittels Sprühdüsen mit einer Wachsemulsion aus 50% Carnaubawachs, und 50% Wasser. Des Weiteren wurden Hilfsstoffe hinzugegeben. Das Messer drehte sich hierbei einmal von einer Seite zur anderen, durch die sich selbst drehenden Zylinder, die gleichzeitig durch das Drehen die Messer nach vorne beförderte und durch darüber angebrachte Ventilatoren trockneten. Zuletzt fielen die Messer in einen Karton und wurden anschließend nach Hygienevorschriften verpackt.

### Beispiel 3: Herstellung eines Lollistiels/Lollisticks

Das Ausgangsmaterial für den Lollistiel/Lollistick bestand aus 52% Maisstärke, 43% Guarkernmehl, 5% Xanthan. Zusätzlich bezogen auf das Nettogewicht bezogen wurden 1% Carnaubawachspulver, 3% Sonnenblumenöl, 2% Lebensmittelfarbe und 40% Wasser hinzugefügt. Diese Rohmaterialen, ausschließlich Wasser, Lebensmittelfarbe und Sonnenblumenöl, wurden in einem Mixer verrührt. Dies geschieht solange bis sich die verschiedenen Bestandteile gut vermischt haben. Daraufhin wurde Wasser (+40% auf das Nettogewicht), Lebensmittelfarbe (+2% auf das Nettogewicht) und Sonnenblumenöl (+3% auf das Nettogewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die pulvrige Mischung wurde dann in einen "Screw Conveyor", welcher die Mischung weiter nach oben transportierte, gegeben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch 40 Auslässe (Matrizen) in ihre Form zu Strängen gepresst. Dadurch bildete sich die gewünschte vorläufige Lollistiel-Form. Die geformten Lollistiele hatten eine Wandstärke von 1 mm und einen Durchmesser von 3 mm. Die Lolli-Stränge wurden anschließend in einer "Cooling und Cutter"-Maschine" bearbeitet. Hierbei trockneten die Lolli-Stränge, bei Durchlaufen des Förderbandes, wo an der Oberseite der Maschine angebrachte Ventilatoren das noch feuchte (35%) und warme (65C°) Material, runter kühlten. Nach durchlaufen des 10 m langen Kühlungstunnels liefen die Stränge in die Schneidemaschine, welche die Stränge in die gewünschte Länge von 20 cm mit einen von oben nach unten stoßendem Klinge zuschnitten. Schließlich wurden die zugeschnittenen Lollistiele auf das Förderband eines Heiztunnels weiterbefördert, welcher die Lollistiele bei circa 90 °C Heißluft und UV-Licht vollständig aushärtete und desinfizierte. Am Ende des Heiztunnels wurden die Lollistiele in eine Beschichtungsmaschine befördert. Diese beschichtete die Lollistiele mittels Sprühdüsen mit einer Emulsion (32,5% Carnaubawachs, 17,5% Natur Latex und 50% Wasser). Des Weiteren wurden Hilfsstoffe und ein oder mehrere anti-stick Zusätze hinzugegeben. Der Lollistiel/Lollistick drehte sich hierbei einmal 360 Grad durch die sich selbst drehenden Zylinder, die gleichzeitig durch das Drehen die Lollistiele/Lollistick nach vorne beförderte. Anschließend wurden der Lollistiel/Lollistick mittels Sprühdüsen ein Glycerin- Film geschaffen. Hierbei drehte sich der Lollistiel/Lollistick um 360° durch die sich selbst drehenden Zylinder. Zuletzt fielen die Lollistiele/Lollisticks in einen Karton und wurden anschließend nach Hygienevorschriften verpackt.

### Beispiel 4: Herstellung Eisstiel

Das Ausgangsmaterial für den Eis-Stiel besteht aus 50% Tapiokastärke, 45% Guarkernmehl, 5% Xanthan. Zusätzlich bezogen auf das Nettogewicht plus 2% Nussöl, 4% Rapswachsemulsion und 30% Wasser. Diese Rohmaterialen, ausschließlich Wasser, Rapswachsemulsion und Öl, wurden in einem Mixer verrührt. Dies geschieht solange bis sich die verschiedenen Bestandteile gut vermischt haben. Daraufhin wurde Wasser (+40% auf das Nettogewicht), Rapswachsemulsion (+4% auf das Nettogewicht) und Nussöl (+2% auf das Nettogewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch pulvrige Mischung wurde dann in einen "Screw Conveyor", welcher die Mischung weiter nach oben transportierte, gegeben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch einen Auslass (Matrize) in ihre Form eines fast schließenden Kreises gepresst. Die Masse öffnet sich und faltet sich aus. Dadurch bildete sich die gewünschte vorläufige Form. Mit einer Wandstärke von 2 mm. Die 50 cm breite Teigplatte wird in den Ausstanzbereich befördert. Hierbei wird die Masse mit einer Walzmaschine zu dem gewünschten der Form, Eis-Stiel aus der flachen (2 mm) Teigplatte, ausgestochen. Dadurch bildete sich die gewünschte Eisstiel-Form. Der geformte Eisstiel weist eine Dicke von 2 mm auf. Schließlich wurde der zugeschnittene Eisstiel auf das Förderband eines Heiztunnels befördert, welcher den Eisstiel bei circa 80°C Heißluft und UV-Licht vollständig aushärtete und desinfizierte. Am Ende des Heiztunnels wurden die Eisstiele in eine Beschichtungsmaschine befördert. Diese beschichtete die Eisstiele mittels Sprühdüsen mit einer Wachsemulsion aus (50% Sojawachs, und 50% Wasser) Des Weiteren wurden Hilfsstoffe hinzugegeben. Der Eisstiel drehte sich hierbei einmal von einer Seite zur anderen, durch die sich selbst drehenden Zylinder, die gleichzeitig durch das Drehen die Eisstiele nach vorne beförderte und durch darüber angebrachte Ventilatoren trockneten. Zuletzt fielen die Eisstiele in einen Karton und wurden anschließend nach Hygienevorschriften verpackt.

### Beispiel 5: Herstellung eines Trinkhalms

Das Ausgangsmaterial für den Trinkhalm bestand aus 55% Weizenstärke, 40% Guarkernmehl, 5% Xanthan. Zusätzlich bezogen auf das Nettogewicht wurden 2% Sojawachspulver, 4% Sonnenblumenöl, 10% Glycerin, 2% Lebensmittelfarbe und 40% Wasser hinzugefügt. Diese Rohmaterialen, ausschließlich Wasser, Lebensmittelfarbe, Glycerin und Sonnenblumenöl, wurden in einem Mixer verrührt. Dies geschieht solange bis sich die verschiedenen Bestandteile gut vermischt haben. Daraufhin wurde Wasser (+40% auf das Nettogewicht), Glycerin (+ 10 auf das Nettogewicht), Lebensmittefarbe (+ 2% auf das Nettogewicht) und Sonnenblumen Öl (+4% auf das Nettogewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch sehr pulvrige Mischung wurde dann in einen "Screw Conveyor", welcher die Mischung weiter nach oben transportierte, geben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch 10 Auslässe (Matrizen) in ihre Form zu Strängen gepresst. Dadurch bildete sich die gewünschte vorläufige Trinkhalm-Form. Die geformten Trinkhalm-Stränge hatten eine Wandstärke von 1 mm und einen Durchmesser von 5 mm. Die Stränge wurden anschließend in einer "Cooling und Cutter"-Maschine bearbeitet. Hierbei trockneten die Stränge, bei Durchlaufen des Förderbandes, wo an der Oberseite der Maschine angebrachte Ventilatoren das noch feuchte (35%) und warme (65°C) Material, abkühlten. Nach Durchlaufen des 10 m langen Kühlungstunnels, liefen die Stränge in die Schneidemaschine, welche die Stränge in die gewünschte Länge von 18 cm mit einen von oben nach unten stoßenden Klinge zuschnitten. Schließlich wurden die zugeschnittenen Trinkhalme auf das Förderband eines Heiztunnels weiterbefördert, welcher die Trinkhalme bei circa 85 °C Heißluft und UV-Licht vollständig aushärtete und desinfizierte. Am Ende des Heiztunnels wurden die Trinkhalme in eine "Beschichtungsmaschine" befördert. Diese beförderte die Trinkhalme mittels einem Förderband mit Lamellen in ein Becken mit einer Emulsion, bestehend aus 5% Rapswachs, 75% Naturlatex und 20% Wasser. Des Weiteren wurden Hilfsstoffe und ein oder mehrere anti-stick Zusätze hinzugegeben. Somit wurden die Trinkhalme sowohl innen, als auch außen beschichtet. Anschließend wurden die Trinkhalme auf einen weiteren Trocknungstunnel befördert wo die Beschichtung aushärtete. Anschließend drehte sich der Trinkhalm hierbei einmal um 360 Grad durch die sich selbst drehenden Zylinder, die gleichzeitig durch das Drehen die Trinkhalme nach vorne befördern und mittels Sprühdüsen mit einen hauchdünnen Schicht Glycerin besprühte. Zuletzt vielen die Trinkhalme in einen Karton und wurden anschließend nach Hygienevorschriften verpackt.

### Beispiel 6: Beschichtung eines Trinkhalms

Die Trinkhalme wurden mittels einem Förderband mit Lamellen in ein Becken mit einer Wachsemulsion, bestehend aus 32,5% Carnaubawachs, 17,5% Paraffin und 50% Wasser befördert. Somit wurden die Trinkhalme sowohl innen, als auch außen beschichtet. Anschließend wurden die Trinkhalme auf einen weiteren Trocknungstunnnel befördert, wo die Beschichtung aushärtete.

Alternativ wurden die Trinkhalme mittels einem Förderband mit Lamellen in ein Becken mit einer Emulsion befördert, wobei diese aus 5% Carnaubawachs und 95 % Kautschukemulsion oder Naturlatexmilch und zusätzlich aus ein oder mehreren anti-stick Zusätzen besteht. Somit wurden die Trinkhalme sowohl innen, als auch außen beschichtet. Anschließend wurden die Trinkhalme auf einen weiteren Trocknungstunnel befördert, wo die Beschichtung aushärtete. Anschließend drehte sich der Trinkhalm hierbei einmal um 360 Grad durch die sich selbst drehenden Zylinder, die gleichzeitig durch das Drehen die Trinkhalme nach vorne beförderte und mittels Sprühdüsen mit einen hauchdünnen Schicht Glycerin besprühte. Zuletzt vielen die Trinkhalme in einen Karton und wurden anschließend nach Hygienevorschriften verpackt.

Alternativ wurden die Trinkhalme mittels einem Förderband mit Lamellen in ein Becken mit einer Emulsion befördert, wobei diese aus 3% Rapswachs, 1% Glycerin, 1% Magnesiumstearat und 95 % Kautschukemulsion oder Naturlatexmilch und zusätzlich aus ein oder mehreren anti-stick Zusätzen besteht. Somit wurden die Trinkhalme sowohl innen, als auch außen beschichtet. Anschließend wurden die Trinkhalme auf einen weiteren Trocknungstunnel befördert, wo die Beschichtung aushärtete. Anschließend drehte sich der Trinkhalm hierbei einmal um 360 Grad durch die sich selbst drehenden Zylinder, die gleichzeitig durch das Drehen die Trinkhalme nach vorne beförderte und in eine "Dust- Box" beförderte und ein Staubfilm Magnesium Stearate auf der Beschichtung legte. Zuletzt vielen die Trinkhalme in einen Karton und wurden anschließend nach Hygienevorschriften verpackt.

### Beispiel 7: Kompostierbarkeit der erfindungsgemäßen Trinkhalme

Die erfindungsgemäßen Trinkhalme wurden in zwei unterschiedlichen Verfahren auf ihre Kompostierbarkeit getestet. In einem ersten Verfahren wurde das Testmaterial durch "Intensivrotte" getestet. Dazu wurde das Material in einen Rottetunnel gebracht. Nach 2 Umsatzvorgängen wurde das Material ausgetragen und abgesiebt. Kontrolliert wurde das Testsäckchen vor dem Absieben. Es befand sich kein Testmaterial mehr im Sack. Das Testmaterial war daher nach 4 Wochen völlig zersetzt und somit kompostiert.

In einem zweiten Verfahren wurde das Testmaterial mittels Trockenvergärung getestet. Das Testmaterial wurde in einen Fermentertunnel gebracht. Nach 27 Tagen wurde der Grad der Kompostierung getestet. Das Material war nach 27 Tagen vollständig aufgelöst und somit kompostiert.

### Offenbarte Gegenstände

1. Hilfsmittel zum Essen oder zum Trinken umfassend
   pflanzliche Stärke und
   pflanzliches Dickungs- oder Geliermittel,
   wobei das Hilfsmittel kompostierbar ist.
2. Hilfsmittel zum Essen oder zum Trinken gemäß Gegenstand 1, wobei das Hilfsmittel gemäß DIN EN 13432, Version 2000-12 kompostierbar ist.
3. Hilfsmittel zum Essen oder zum Trinken gemäß Gegenstand 1 oder 2, wobei das Hilfsmittel innerhalb von 50 Tagen biologisch abbaubar ist.
4. Hilfsmittel zum Essen oder zum Trinken gemäß einem der Gegenstände 1-3, ferner umfassend Zellstoff und/oder Holzstoff.
5. Hilfsmittel zum Essen oder zum Trinken gemäß einem der Gegenstände 1-4, ferner umfassend Wachs, bevorzugt Carnaubawachs oder Sojawachs.
6. Hilfsmittel zum Essen oder zum Trinken gemäß einem der Gegenstände 1-5, ferner umfassend Öl, bevorzugt Nussöl oder Sonnenblumenöl.
7. Hilfsmittel zum Essen oder zum Trinken gemäß einem der Gegenstände 1-6, ferner umfassend eine äußere Beschichtung umfassend Carnaubawachs, und/oder Rapswachs, und/oder Paraffin, oder Mischungen davon, vorzugsweise ferner umfassend Biopolymere, insbesondere Naturlatex.
8. Hilfsmittel zum Essen oder zum Trinken gemäß Gegenstand 7, wobei die äußere Beschichtung Hilfsstoffe und/oder anti-sick Zusätze umfasst.
9. Hilfsmittel zum Essen oder zum Trinken gemäß einem der vorherigen Gegenstände, wobei die pflanzliche Stärke ausgewählt wird aus der Gruppe bestehend aus Weizenstärke, Kartoffelstärke, Maisstärke, Tapiokastärke oder eine Mischung davon ist und wobei das pflanzliche Dickungs- oder Geliermittel eine Mischung aus Guarkernmehl und Xanthan ist.
10. Hilfsmittel zum Essen oder zum Trinken gemäß einem der vorherigen Gegenstände, wobei das Hilfsmittel
   40-50% pflanzliche Stärke,
   35-45% Guarkernmehl und
   1-5% Xanthan und optiopional
   eine Wachs- und/oder Naturlatex-basierte Beschichtung umfasst.
11. Hilfsmittel zum Essen oder zum Trinken gemäß einem der Gegenstände 1-10, wobei Glycerin als eine zweite Beschichtung auf das Hilfsmittel aufgesprüht wird.
12. Hilfsmittel zum Essen oder zum Trinken gemäß einem der Gegenstände 1-11, wobei Glycerin als eine zweite Beschichtung auf das Hilfsmittel aufgesprüht wird.
13. Hilfsmittel zum Essen oder zum Trinken gemäß einem der Gegenstände 1-12, wobei das Hilfsmittel ein Trinkhalm, ein Becher, ein Besteck oder ein Lollistiel ist.
14. Hilfsmittel zum Essen oder zum Trinken gemäß einem der Gegenstände 1-12 umfassend
   40-50% Stärke,
   35-45% Guarkernmehl,
   1-5% Xanthan,
   Farbstoff, und eine
   Naturlatex-basierte Beschichtung,
   wobei die Beschichtung aus 5% Carnaubawachs, 2% Glycerin, 93% Naturlatexmilch besteht und
   wobei das Hilfsmittel ein kompostierbarer Trinkhalm ist.
15. Medizinischer Bedarfsartikel umfassend
   pflanzliche Stärke und
   pflanzliches Dickungs- oder Geliermittel,
   wobei der medizinische Bedarfsartikel kompostierbar ist.
16. Medizinischer Bedarfsartikel gemäß Gegenstand 15, wobei das Hilfsmittel gemäß DIN EN 13432, Version 2000-12 kompostierbar ist.
17. Medizinischer Bedarfsartikel gemäß Gegenstand 15 oder 16, wobei das Hilfsmittel innerhalb von 50 Tagen biologisch abbaubar ist.
18. Medizinischer Bedarfsartikel gemäß einem der Gegenstände 15-17, ferner umfassend Zellstoff und/oder Holzstoff.
19. Medizinischer Bedarfsartikel gemäß einem der Gegenstände 15-18, ferner umfassend Wachs, bevorzugt Carnaubawachs oder Sojawachs.
20. Medizinischer Bedarfsartikel gemäß einem der vorherigen Gegenstände, ferner umfassend Öl, bevorzugt Nussöl oder Sonnenblumenöl.
21. Medizinischer Bedarfsartikel gemäß einem der vorherigen Gegenstände, ferner umfassend Glycerin.
22. Medizinischer Bedarfsartikel gemäß einem der vorherigen Gegenstände, ferner umfassend eine äußere Beschichtung umfassend Carnaubawachs, und/oder Rapswachs, und/oder Paraffin, oder Mischungen davon, vorzugsweise umfassend Biopolymere, insbesondere Naturlatex.
23. Medizinischer Bedarfsartikel gemäß einem der vorherigen Gegenstände, wobei die pflanzliche Stärke ausgewählt wird aus der Gruppe bestehend aus Weizenstärke, Kartoffelstärke, Maisstärke, Tapiokastärke oder eine Mischung davon ist und wobei das pflanzliche Dickungs- oder Geliermittel eine Mischung aus Guarkernmehl und Xanthan ist.
24. Medizinischer Bedarfsartikel gemäß einem der vorherigen Gegenstände, wobei der Bedarfsartikel
   40-50% pflanzliche Stärke,
   35-45% Guarkernmehl und
   1-5% Xanthan und optiopional
   eine Wachs-basierte Beschichtung umfasst.
25. Medizinischer Bedarfsartikel gemäß einem der vorherigen Gegenstände, wobei der Bedarfsartikel ein Wattestäbchen, ein Mundspatel oder eine Einmalzahnbürste ist.
26. Verfahren zur Herstellung von kompostierbaren Hilfsmitteln zum Essen oder Trinken oder kompostierbaren medizinischen Bedarfsartikeln, wobei das Verfahren die folgenden Schritte umfasst:
   a. Herstellen einer Mischung, die pflanzliche Stärke und pflanzliches Dickungs- oder Geliermittel umfasst, umfassend die Schritte
      a.1 Zusammenfügen von festen Bestandteilen der Mischung umfassend pflanzliche Stärke und pflanzliches Dickungs- und Geliermittel und Mischen dieser Bestandteile,
      a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
   b. Formen der Mischung aus Schritt a. zu einem Hilfsmittel zum Essen oder Trinken oder zu einem medizinischen Bedarfsartikel,
   c. Aushärten des in Schritt b. geformten Hilfsmittels zum Essen oder Trinken oder medizinischen Bedarfsartikels,
   d. optional, Beschichten des in Schritt c. ausgehärteten Hilfsmittels zum Essen oder Trinken oder medizinischen Bedarfsartikels mit einer Emulsion umfassend Carnaubawachs, und/oder Rapswachs, und/oder Paraffin, oder Mischungen davon, vorzugsweise ferner umfassend Biopolymere, insbesondere Naturlatex.
27. Verfahren zur Herstellung von kompostierbaren Hilfsmitteln zum Essen oder Trinken oder kompostierbaren medizinischen Bedarfsartikeln gemäß Gegenstand 26, wobei das Verfahren die folgenden Schritte umfasst:
   a. Herstellen einer Mischung, die pflanzliche Stärke und pflanzliches Dickungs- oder Geliermittel umfasst, umfassend die Schritte
      a.1 Zusammenfügen von festen Bestandteilen der Mischung umfassend
         50-70% pflanzliche Stärke und
         30-50% pflanzliches Dickungs- und Geliermittel
         und Mischen dieser Bestandteile,
      a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile.
   b. Formen der Mischung aus Schritt a. zu einem Hilfsmittel zum Essen oder Trinken oder einem medizinischen Bedarfsartikel,
   c. Aushärten des in Schritt b. geformten Hilfsmittels zum Essen oder Trinken oder medizinischen Bedarfsartikels,
   d. optional, Beschichten des in Schritt c. ausgehärteten Hilfsmittels zum Essen oder Trinken oder medizinischen Bedarfsartikels mit einer Wachsemulsion umfassend Carnaubawachs, und/oder Rapswachs, und/oder Paraffin, oder Mischungen davon, vorzugsweise umfassend Biopolymere, insbesondere Naturlatex.
28. Verfahren zur Herstellung von kompostierbaren Hilfsmitteln zum Essen oder Trinken oder kompostierbaren medizinischen Bedarfsartikeln gemäß einem der Gegenstände 26 und 27 wobei die pflanzliche Stärke in der Mischung in Schritt a.1 Weizenstärke, Kartoffelstärke, Maisstärke, Tapiokastärke oder eine Mischung davon ist, und wobei das pflanzliche Dickungs- und Geliermittel in der Mischung in Schritt a.1 eine Mischung aus Guarkernmehl und Xanthan ist.
29. Verfahren zur Herstellung von kompostierbaren Hilfsmitteln zum Essen oder Trinken oder kompostierbaren medizinischen Bedarfsartikeln gemäß einem der Gegenstände 26-28, wobei die festen Bestandteile aus Schritt a.1 ferner Zellstoff und/oder Holzstoff und/oder Wachspulver umfassen.
30. Verfahren zur Herstellung von kompostierbaren Hilfsmitteln zum Essen oder Trinken oder kompostierbaren medizinischen Bedarfsartikeln gemäß einem der Gegenstände 26-29, wobei die flüssigen Bestandteile aus Schritt a.2 ferner Öl, bevorzugt Nussöl oder Sonnenblumenöl, Glycerin und/oder eine Wachsemulsion umfassen.
31. Verfahren zur Herstellung von kompostierbaren Hilfsmitteln zum Essen oder Trinken oder kompostierbaren medizinischen Bedarfsartikeln gemäß einem der Gegenstände 26-30, wobei das Verfahren die folgenden Schritte umfasst:
   a. Herstellen einer Mischung, die pflanzliche Stärke und pflanzliches Dickungs- oder Geliermittel umfasst, umfassend die Schritte
      a.1 Zusammenfügen von festen Bestandteilen der Mischung enthaltend
         50% Weizenstärke
         45% Guarkernmehl
         5% Xanthan
         und Mischen dieser Bestandteile,
      a.2 Zugabe von flüssigen Bestandteilen der Mischung zur Mischung aus Schritt a.1 umfassend bezogen auf das Nettogewicht der Mischung aus Schritt a.1
         2% Carnaubawachsemulsion
         4% Sonnenblumenöl
         5% Glycerin und
         40% Wasser
         und Mischen dieser Bestandteile
   b. Formen der Mischung aus Schritt a. zu einem Hilfsmittel zum Essen oder Trinken oder einem medizinischen Bedarfsartikel,
   c. Aushärten des in Schritt b. geformten Hilfsmittels zum Essen oder Trinken oder medizinischen Bedarfsartikels,
   d. optional, Beschichten des in Schritt c. ausgehärteten Hilfsmittels zum Essen oder Trinken oder medizinischen Bedarfsartikels mit einer Emulsion.
32. Verfahren zur Herstellung von kompostierbaren Hilfsmitteln zum Essen oder Trinken oder kompostierbaren medizinischen Bedarfsartikeln gemäß einem der Gegenstände 26-31, wobei die Emulsion zur Beschichtung im optionalen Schritt d. entweder aus 32,5% Carnaubawachs, 17,5% Naturlatexmilch und 50% Wasser oder aus 50% Carnaubawachs und 50% Wasser besteht, oder aus 5 % Carnaubawachs und 95% Naturlatex.

## Patentansprüche

1. Medizinischer Bedarfsartikel umfassend
pflanzliche Stärke und
pflanzliches Dickungs- oder Geliermittel,
wobei der medizinische Bedarfsartikel kompostierbar ist und wobei der medizinische Bedarfsartikel
40-50% pflanzliche Stärke,
35-50% Dickungs- oder Geliermittel
0-10% Zellstoff und/oder Holzstoff
0-10% Wachs, bevorzugt Carnaubawachs oder Sojawachs
0-3% Nussöl oder Sonnenblumenöl
0-2% Glycerin und
optional eine äußere Beschichtung umfasst.

2. Medizinischer Bedarfsartikel gemäß Anspruch 1, ferner umfassend eine äußere Beschichtung umfassend Carnaubawachs, und/oder Rapswachs, und/oder Paraffin, oder Mischungen davon, vorzugsweise ferner umfassend Biopolymere, insbesondere Naturlatex.

3. Medizinischer Bedarfsartikel gemäß einem der Ansprüche 1 oder 2, wobei der medizinische Bedarfsartikel
40-50% pflanzliche Stärke,
35-45% Guarkernmehl und
1-5% Xanthan und optiopional
eine Wachs- und/oder Naturlatex-basierte Beschichtung umfasst.

4. Medizinischer Bedarfsartikel gemäß einem der Ansprüche 1-3, wobei die pflanzliche Stärke ausgewählt wird aus der Gruppe bestehend aus Weizenstärke, Kartoffelstärke, Maisstärke, Tapiokastärke oder eine Mischung davon ist und wobei das pflanzliche Dickungs- oder Geliermittel eine Mischung aus Guarkernmehl und Xanthan ist.

5. Medizinischer Bedarfsartikel gemäß einem der Ansprüche 1-4, wobei der medizinische Bedarfsartikel
40-50% pflanzliche Stärke,
35-45% Guarkernmehl,
1-5% Xanthan, und optional
eine Wachs-basierte Beschichtung umfasst.

6. Medizinischer Bedarfsartikel gemäß einem der Ansprüche 1-3, wobei der medizinische Bedarfsartikel ein Wattestäbchen, ein Mundspatel oder eine Einmalzahnbürste ist.

7. Verfahren zur Herstellung von kompostierbaren medizinischen Bedarfsartikeln nach einem der Ansprüche 1-6, wobei das Verfahren die folgenden Schritte umfasst:
a. Herstellen einer Mischung, die pflanzliche Stärke und pflanzliches Dickungs- oder Geliermittel umfasst, umfassend die Schritte
a.1 Zusammenfügen von festen Bestandteilen der Mischung umfassend pflanzliche Stärke und pflanzliches Dickungs- und Geliermittel und Mischen dieser Bestandteile,
a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu einem medizinischen Bedarfsartikel, wobei die Mischung von a. vor dem Formen mittels einer Plastifizierschnecke verdichtet wird,
c. Aushärten des in Schritt b. geformten medizinischen Bedarfsartikels,
d. optional, Beschichten des in Schritt c. ausgehärteten medizinischen Bedarfsartikels mit einer Emulsion umfassend Carnaubawachs, und/oder Rapswachs, und/oder Paraffin, oder Mischungen davon, vorzugsweise ferner umfassend Biopolymere, insbesondere Naturlatex.

8. Verfahren nach Anspruch 7, wobei die Emulsion zur Beschichtung im optionalen Schritt d. entweder aus 32,5% Carnaubawachs, 17,5% Naturlatexmilch und 50% Wasser oder aus 50% Carnaubawachs und 50% Wasser besteht, oder aus 5 % Carnaubawachs und 95% Naturlatex.
